(19) Europäisches Patentamt
European Patent Office
Office européen des brevets

(11) EP 1 149 835 A1

(12) EUROPEAN PATENT APPLICATION
published in accordance with Art. 158(3) EPC

(43) Date of publication:
**31.10.2001 Bulletin 2001/44**

(21) Application number: **00901971.2**

(22) Date of filing: **28.01.2000**

(51) Int Cl.[7]: **C07D 487/04**, A61K 31/519, A61P 3/04, A61P 3/06, A61P 3/10, C12N 5/06, C12Q 1/02

(86) International application number:
**PCT/JP00/00462**

(87) International publication number:
**WO 00/44754 (03.08.2000 Gazette 2000/31)**

(84) Designated Contracting States:
**AT BE CH CY DE DK ES FI FR GB GR IE IT LI LU MC NL PT SE**
Designated Extension States:
**AL LT LV MK RO SI**

(30) Priority: **29.01.1999 JP 2235799**

(71) Applicants:
- **SUMITOMO CHEMICAL COMPANY LIMITED**
  **Osaka-shi, Osaka 541-0041 (JP)**
- **Sumitomo Pharmaceuticals Company, Limited**
  **Osaka 541-8510 (JP)**

(72) Inventors:
- **OHTSUBO, Tsuguteru**
  **Toyonaka-shi, Osaka 561-0802 (JP)**
- **MURAKAMI, Hiroko**
  **Ashiya-shi, Hyogo 659-0012 (JP)**

(74) Representative: **Cresswell, Thomas Anthony et al**
**J.A. KEMP & CO.**
**14 South Square**
**Gray's Inn**
**London WC1R 5JJ (GB)**

(54) **FAT ACCUMULATION INHIBITORY AGENTS**

(57) Aminopyrimidine derivatives represented by general formula:

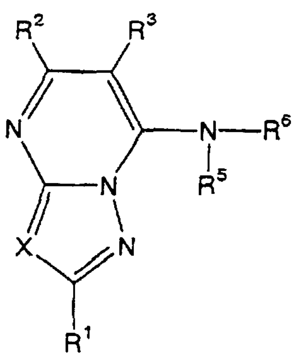

wherein $R^1$ is a hydrogen atom, an alkyl group, an alkenyl group, etc.; $R^2$ and $R^3$ are a hydrogen atom, a halogen atom, an alkyl group, an alkenyl group, etc.; $R^5$ is a hydrogen atom, an alkyl group, etc.; $R^6$ is a $C_1$ to $C_{12}$ alkyl group, a $C_2$-$C_{12}$ alkenyl group, etc.; X is a nitrogen atom, CH, etc., can suppress the accumulation of fat in fat cells and are thus effective for the prophylaxis and treatment of various disorders accompanied by increased adipose tissues.

EP 1 149 835 A1

**Description**

TECHNICAL FIELD

The present invention relates to a pharmaceutical composition for suppressing the accumulation of fat comprising as an active ingredient an aminopyrimidine derivative, and novel aminopyrimidine derivatives, a method for assaying a fat accumulation suppressing activity of a test substance in fat cells, as well as a method for preparing a mature adipocyte population useful for the assay method, etc. The fat accumulation suppressing agent of the present invention is useful for the prevention and treatment of obesity accompanied by an increase in body fat or an increase of adipose tissues in the abdominal cavity and various diseases such as diabetes mellitus, hyperglycemia, etc.

BACKGROUND ART

Fat cells have the function to store fat in their cells. In the body of a mammal including human, fat cells are typically present in the adipose tissues of the subcutaneous abdominal region, the femoral region, the gluteal region, the pectoral region, etc. and in the adipose tissues which are in the abdominal cavity in the vicinity of the mesenterium, kidney, epididymis, etc. It is known that accumulation of fat in fat cells results in, for example, obesity accompanied by an increase in body fat and an increase in adipose tissues in the abdominal cavity, and thus further induces disorders such as impairment in glucose tolerance [Journal of Clinical Investigation, vol. 72, p. 1150 (1983)], diabetes mellitus [National Diabetes Data Group: Diabetes in America. Bethesda, MD., U.S. Dept. of Health and Human Services, (1985), Diabetes Care, vol. 19, p. 613 (1996), Diabete & Metabolisme, vol. 20, p. 375 (1994), Obesity: Advances in Understanding and Treatment, Published by IBC Biomedical Library, Chapter 3.1, (1996)], hyperglycemia, hyperlipidemia, hypertension [Journal of Clinical Investigation, vol. 72, p. 1150 (1983)], coronary arterial diseases [Diabete & Metabolisme, vol. 20, p. 375 (1994)], obstructive arterial sclerosis and the like [WHO Expert Committee on Diabetes Mellitus. Second report, WHO Tech Rep 646 Geneva: World Health Organization (1980)].

Therefore, there is a strong demand for the development of drugs that are useful for the prevention and treatment of various diseases accompanied by increased adipose tissues, by suppressing accumulation of fat in fat cells and thus preventing an increase of adipose tissues.

DISCLOSURE OF INVENTION

Under the foregoing situation, the present inventors have made extensive investigations and as a result, have found a method for efficiently screening a substance having an activity of suppressing the accumulation of fat in fat cells. They have further found that some aminopyrimidine derivatives can suppress the accumulation of fat in fat cells. The present invention has thus been accomplished.

That is, the present invention relates to (1) through (32) below:

(1) a pharmaceutical composition for suppressing the accumulation of fat comprising as an active ingredient an aminopyrimidine derivative represented by general formula (I):

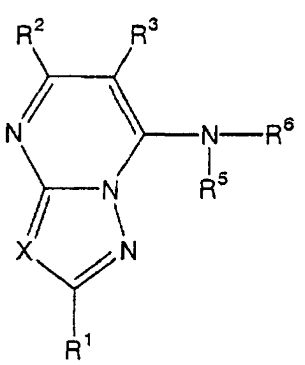

(I)

wherein:

$R^1$ is a hydrogen atom, an alkyl group which may be substituted, an alkenyl group which may be substituted,

an aryl group which may be substituted, an aralkyl group which may be substituted, or a heterocyclic group which may be substituted;
$R^2$ and $R^3$, which may be the same or different, represent a hydrogen atom, a halogen atom, an alkyl group which may be substituted, an alkenyl group which may be substituted, an aryl group which may be substituted, an aralkyl group which may be substituted, or a heterocyclic group which may be substituted; or $R^2$ and $R^3$ may be combined together to form a C3 to C10 alkylene group which may be substituted;
X is a nitrogen atom or a group of C-$R^4$ wherein $R^4$ is a hydrogen atom, a halogen atom, an alkyl group which may be substituted, an alkenyl group which may be substituted, an aryl group which may be substituted, or an aralkyl group which may be substituted;
$R^5$ is a hydrogen atom, an alkyl group which may be substituted, or an alkenyl group which may be substituted;
$R^6$ is a C1 to C12 alkyl group which may be substituted, a C2-C12 alkenyl group which may be substituted, an acyl group, or a group of general formula (II):

-A-Y-B-Z (II)

wherein:
A is a carbonyl group or a single bond;
Y is an alkylene group which may be substituted;
B is an oxygen atom or a single bond; and
Z is an aryl group which may be substituted or a heterocyclic group which may be substituted; or a pharmacologically acceptable salt thereof;

(2) a pharmaceutical composition for suppressing the accumulation of fat according to (1), wherein in the general formula (I):

$R^6$ is a C1 to C12 alkyl group which may be substituted, a C2-C12 alkenyl group which may be substituted, or a group of general formula (II):

-A-Y-B-Z (II)

wherein:
A is a single bond;
Y is an alkylene group which may be substituted;
B is an oxygen atom or a single bond; and
Z is an aryl group which may be substituted or a heterocyclic group which may be substituted;

(3) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is an oxygen atom; and
Z is an aryl group which may be substituted or a heterocyclic group which may be substituted;

(4) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is a single bond; and
Z is an aryl group which may be substituted or a heterocyclic group which may be substituted;

(5) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is an oxygen atom; and
Z is an aryl group which may be substituted or a heterocyclic group which may be substituted;

(6) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is a single bond; and
Z is an aryl group which may be substituted or a heterocyclic group which may be substituted;

(7) a pharmaceutical composition for suppressing the accumulation of fat according to (1), wherein in the general formula (I):

$R^6$ is a C1 to C12 alkyl group which may be substituted, an C2 to C12 alkenyl group which may be substituted, or a group of general formula (II):

$$-A-Y-B-Z \quad (II)$$

wherein:
A is a carbonyl group;
Y is an alkylene group which may be substituted;
B is an oxygen atom or a single bond; and
Z is an aryl group which may be substituted or a heterocyclic group which may be substituted;

(8) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a carbonyl group;
Y is an alkylene group which may be substituted;
B is an oxygen atom; and
Z is an aryl group which may be substituted or a heterocyclic group which may be substituted;

(9) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a carbonyl group;
Y is an alkylene group which may be substituted;
B is a single bond; and
Z is an aryl group which may be substituted or a heterocyclic group which may be substituted;

(10) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a carbonyl group;
Y is an alkylene group which may be substituted;
B is an oxygen atom; and
Z is an aryl group which may be substituted or a heterocyclic group which may be substituted;

(11) a pharmaceutical composition for suppressing the accumulation of fat according to (1), wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a carbonyl group;
Y is an alkylene group which may be substituted;
B is a single bond; and
Z is an aryl group which may be substituted or a heterocyclic group which may be substituted;

(12) a pharmaceutical composition for suppressing the accumulation of fat according to (1), comprising an aminopyrimidine derivative selected from the group consisting of the compounds below, or a pharmacologically acceptable salt thereof:

N-[2-(2,4-dimethylphenoxy)ethyl]-5,6-dimethyl[1,2,4]-triazolo[1,5-a]pyrimidin-7-amine;
N-{3-[4-(tert-butyl)phenoxy]propyl}-5,6-dimethyl-[1,2,4]triazolo[1,5-a]pyrimidin-7-amine; 5,6-dimethyl-N-{2-[4-(1-methyl-1-phenylethyl)phenoxy]-ethyl}[1,2,4]triazolo[1,5-a]pyrimidin-7-amine;
N-[2-{2,3-dimethyl-4-[4-(methylsulfanyl)phenoxy]-phenoxy}ethyl]-5,6-dimethyl[1,2,4]triazolo[1,5-a]pyrimidin-7-amine;
N-{2-[4-(2-chloro-4-fluorobenzyl)phenoxy]ethyl}-5,6-dimethyl[1,2,4]triazolo[1,5-a]pyrimidin-7-amine;
5,6-dimethyl-N-[2-(2-methyl-4-phenoxyphenoxy)ethyl]-[1,2,4]triazolo[1,5-a]pyrimidin-7-amine;
N-[2-(4-benzyl-2-ethylphenoxy)ethyl]-5,6-dimethyl-[1,2,4]triazolo[1,5-a]pyrimidin-7-amine;
N-[2-(2,4-dimethylphenoxy)ethyl]-5,6,7,8-tetrahydro-[1,2,4]triazolo[5,1-b]quinazolin-9-amine;
N-[2-([1,1'-biphenyl]-4-yloxy)ethyl]-5,6-dimethyl-[1,2,4]triazolo[1,5-a]pyrimidin-7-amine;
N-{2-[4-(2,4-dichlorobenzyl)phenoxy]ethyl}-5,6-dimethyl[1,2,4]triazolo[1,5-a]pyrimidin-7-amine;
N-{2-[4-(benzyloxy)phenoxy]ethyl}-5,6-dimethyl[1,2,4]-triazolo[1,5-a]pyrimidin-7-amine;
N-{2-[4-(3,4-dimethylbenzyl)phenoxy]ethyl}-5,6-dimethyl[1,2,4]triazolo[1,5-a]pyrimidin-7-amine; 5-methyl-N-[2-4(phenoxyphenoxy)ethyl][1,2,4]triazolo-[1,5-a]pyrimidin-7-amine;
N-{2-[4-(tert-butyl)phenoxy]ethyl}-5,6-dimethyl-[1,2,4]triazolo[1,5-a]pyrimidin-7-amine;
5,6-dimethyl-N-[2-(4-phenoxyphenoxy)ethyl]-[1,2,4]-triazolo[1,5-a]pyrimidin-7-amine;
N-[2-(4-benzylphenoxy)ethyl]-5,6-dimethyl[1,2,4]-triazolo[1,5-a]pyrimidin-7-amine;
5,6-dimethyl-N-[2-(1-naphthyloxy)ethyl] [1,2,4]triazolo-[1,5-a]pyrimidin-7-amine;
5,6-dimethyl-N-[2-(2-naphthyloxy)ethyl] [1,2,4]triazolo-[1,5-a]pyrimidin-7-amine;
N-[3-(4-benzylphenoxy)propyl]-5,6-dimethyl[1,2,4]-triazolo[1,5-a]pyrimidin-7-amine;
N-[2-(2-bromo-4-phenoxyphenoxy)ethyl]-5,6-dimethyl-[1,2,4]triazolo[1,5-a]pyrimidin-7-amine;
N-[2-(2-chloro-4-phenoxyphenoxy)ethyl]-5,6-dimethyl-[1,2,4]triazolo[1,5-a]pyrimidin-7-amine;
N-[2-(4-benzyl-2-chlorophenoxy)ethyl]-5,6-dimethyl-[1,2,4]triazolo[1,5-a]pyrimidin-7-amine;
N-[2-(2,6-dimethyl-4-phenoxyphenoxy)ethyl]-5,6-dimethyl[1,2,4]triazolo[1,5-a]pyrimidin-7-amine;
N-(2-{2,6-dimethyl-4-[4-(methylsulfanyl)phenoxy]-phenoxy}ethyl)-5,6-dimethyl[1,2,4]triazolo[1,5-a]pyrimidin-7-amine;
N-{2-[4-(2,4-dimethylphenoxy)-2,6-dimethylphenoxy]-ethyl}-5,6-dimethyl[1,2,4]triazolo[1,5-a]pyrimidin-7-amine;
5,6-dimethyl-N-(2-{4-[4-(methylsulfanyl)phenoxy]-phenoxy}ethyl)[1,2,4]triazolo[1,5-a]pyrimidin-7-amine;
N-[2-(2-fluoro-4-phenoxyphenoxy)ethyl]-5,6-dimethyl-[1,2,4]triazolo[1,5-a]pyrimidin-7-amine;
N-[2-(2,3-dimethyl-4-phenoxyphenoxy)ethyl]-5,6-dimethyl[1,2,4]triazolo[1,5-a]pyrimidin-7-amine;
5,6-dimethyl-N-{2-[4-(phenylsulfanyl)phenoxy]ethyl}-[1,2,4]triazolo[1,5-a]pyrimidin-7-amine;
N-[2-(4-benzyl-2,3-dimethylphenoxy)ethyl]-5,6-dimethyl-[1,2,4]triazolo[1,5-a]pyrimidin-7-amine;
5,6-dimethyl-N-{2-[4-(4-methylbenzyl)phenoxy]ethyl}-[1,2,4]triazolo[1,5-a]pyrimidin-7-amine;
N-{2-[4-(2-fluorobenzyl)phenoxy]ethyl}-5,6-dimethyl-[1,2,4]triazolo[1,5-a]pyrimidin-7-amine;
5,6-dimethyl-N-(2-{4-[4-(methylsulfanyl)benzyl]-phenoxy}ethyl)[1,2,4]triazolo[1,5-a]pyrimidin-7-amine;
5,6-dimethyl-N-{2-[4-(1-phenylethyl)phenoxy]ethyl}-[1,2,4]triazolo[1,5-a]pyrimidin-7-amine;
N-[2-(4-benzyl-2-methylphenoxy)ethyl]-5,6-dimethyl-[1,2,4]triazolo[1,5-a]pyrimidin-7-amine;
N-[2-(4-benzylphenoxy)ethyl]-5-methyl[1,2,4]triazolo-[1,5-a]pyrimidin-7-amine;
N-[2-(4-benzyl-2-methylphenoxy)ethyl]-5-methyl[1,2,4]-triazolo[1,5-a]pyrimidin-7-amine;
N-[2-(2-methyl-4-phenoxyphenoxy)ethyl]-N-(5-methyl-[1,2,4]triazolo[1,5-a]pyrimidin-7-yl)amine;
N-[2-(4-benzyl-2-methylphenoxy)ethyl]-5-ethyl[1,2,4]-triazolo[1,5-a]pyrimidin-7-amine;
N-[2-(4-benzyl-2-ethylphenoxy)ethyl]-5-methyl[1,2,4]-triazolo[1,5-a]pyrimidin-7-amine;
N-[2-(4-benzyl-2,6-dimethylphenoxy)ethyl]-5,6-dimethyl-[1,2,4]triazolo[1,5-a]pyrimidin-7-amine;
N-[2-(4-benzyl-2,6-dimethylphenoxy)ethyl]-5-methyl-[1,2,4]triazolo[1,5-a]pyrimidin-7-amine;
N-[2-(4-benzyl-2-methylphenoxy)ethyl][1,2,4]triazolo-[1,5-a]pyrimidin-7-amine;
5,6-dimethyl-N-(3-phenoxyphenethyl)[1,2,4]triazolo-[1,5-a]pyrimidin-7-amine;
2,5-dimethyl-N-(3-methylphenyl)[1,2,4]triazolo[1,5-a]-pyrimidin-7-amine;
2,5-dimethyl-N-(3-methylbenzyl)[1,2,4]triazolo[1,5-a]-pyrimidin-7-amine;
N-[1-(1-benzothiophen-2-yl)ethyl]-5,6-dimethyl-[1,2,4]triazolo[1,5-a]pyrimidin-7-amine;
5,6-dimethyl-N-{4-[4-(methylsulfanyl)phenoxy]benzyl}-[1,2,4]triazolo[1,5-a]pyrimidin-7-amine;
5,6-dimethyl-N-{4-[4-(methylsulfinyl)phenoxy]benzyl}-[1,2,4]triazolo[1,5-a]pyrimidin-7-amine;
5,6-dimethyl-N-{4-[4-(methylsulfonyl)phenoxy]benzyl}-[1,2,4]triazolo[1,5-a]pyrimidin-7-amine;
N-[2-(2,4-dimethylphenoxy)ethyl]-5,6-dimethylpyrazolo-[1,5-a]pyrimidin-7-amine;
N-{2-[4-(tert-butyl)phenoxy]ethyl}-5,6-dimethyl-pyrazolo[1,5-a]pyrimidin-7-amine;
N-[2-(4-benzyl-2-methylphenoxy)ethyl]-5,6-dimethyl-pyrazolo[1,5-a]pyrimidin-7-amine;
5,6-dimethyl-N-[2-(2-methyl-4-phenoxyphenoxy)ethyl]-pyrazolo[1,5-a]pyrimidin-7-amine;
N-[2-(4-benzyl-2,3-dimethylphenoxy)ethyl]-5,6-dimethyl-pyrazolo[1,5-a]pyrimidin-7-amine;
N-[2-(4-benzyl-2-methylphenoxy)ethyl]pyrazolo[1,5-a]-pyrimidin-7-amine;
N-[2-(4-benzylphenoxy)ethyl]-2-(tert-butyl)-5-methylpyrazolo[1,5-a]pyrimidin-7-amine;
2-(tert-butyl)-N-(2,3-dimethoxybenzyl)-5-methylpyrazolo[1,5-a]pyrimidin-7-amine;
3-hydroxy-N-(2-methylpyrazolo[1,5-a]pyrimidin-7-yl)benzamide;
N-[2-(tert-butyl)pyrazolo[1,5-a]pyrimidin-7-yl]-2,6-dimethoxybenzamide;
2-(tert-butyl)-N-(2-methoxyphenethyl)-5-methylpyrazolo-[1,5-a]pyrimidin-7-amine;
2-(tert-butyl)-N-(3-methoxyphenethyl)-5-methylpyrazolo-[1,5-a]pyrimidin-7-amine;
2-(tert-butyl)-5-methyl-N-phenethylpyrazolo[1,5-a]-pyrimidin-7-amine;
2-(tert-butyl)-5-methyl-N-(3-phenylpropyl)pyrazolo-[1,5-a]pyrimidin-7-amine;
N-[2-(tert-butyl)-5-methylpyrazolo[1,5-a]pyrimidin-7-yl]-N-[4-(3,5-dimethoxyphenoxy)benzyl]amine;
N-[2-(tert-butyl)-5-methylpyrazolo[1,5-a]pyrimidin-7-yl]-N-[4-(3,5-dichlorophenoxy)benzyl]amine;
2-(tert-butyl)-N-(2-methoxybenzyl)-5-methylpyrazolo-[1,5-a]pyrimidin-7-amine;
2-(tert-butyl)-N-(4-methoxyphenethyl)-5-methylpyrazolo-[1,5-a]pyrimidin-7-amine;
2-(tert-butyl)-5-methyl-N-(4-pyridylmethyl)pyrazolo-[1,5-a]pyrimidin-7-amine;
2-(tert-butyl)-5-methyl-N-(4-phenylbutyl)pyrazolo-[1,5-a]pyrimidin-7-amine;
2-(tert-butyl)-5-methyl-N-(2-pyridylmethyl)pyrazolo-[1,5-a]pyrimidin-7-amine; and
2-(tert-butyl)-5-methyl-N-(3-pyridylmethyl)pyrazolo-1,5-a]pyrimidin-7-amine;

(13) an aminopyrimidine derivative represented by general formula (I):

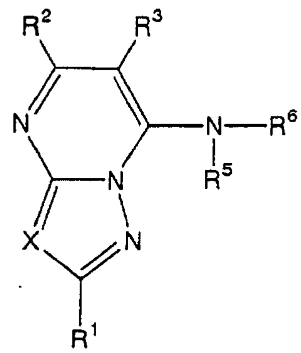

(I)

wherein:

$R^1$ is a hydrogen atom, an alkyl group which may be substituted, an alkenyl group which may be substituted, an aryl group which may be substituted, an aralkyl group which may be substituted, or a heterocyclic group which may be substituted;
$R^2$ and $R^3$, which may be the same or different, represent a hydrogen atom, a halogen atom, an alkyl group which may be substituted, an alkenyl group which may be substituted, an aryl group which may be substituted, an aralkyl group which may be substituted, or a heterocyclic group which may be substituted; or $R^2$ and $R^3$ may be combined together to form a C3 to C10 alkylene group which may be substituted;
X is a nitrogen atom or a group of C-$R^4$ wherein $R^4$ is a hydrogen atom, a halogen atom, an alkyl group which may be substituted, an alkenyl group which may be substituted, an aryl group which may be substituted, or an aralkyl group which may be substituted;
$R^5$ is a hydrogen atom, an alkyl group which may be substituted, or an alkenyl group which may be substituted;
$R^6$ is a group of general formula (II):

-A-Y-B-Z (II)

wherein:
A is a carbonyl group or a single bond;
Y is an alkylene group which may be substituted;
B is an oxygen atom or a single bond; and
Z is an aryl group which may be substituted or a heterocyclic group which may be substituted; provided that when A is a single bond and B is a single bond, Z is a substituted aryl group or a substituted heterocyclic group and the substituent for these groups of Z is a group of formula:

-G-E

wherein G is an oxygen atom or an alkylene group which may be substituted and E is an aryl group which may be substituted or a heterocyclic group which may be substituted;
provided that when $R^1$, $R^3$, $R^4$ and $R^5$ are hydrogen atoms, $R^2$ is a n-butyl group and A is a carbonyl group, Z is a heterocyclic group which may be substituted; or a pharmacologically acceptable salt thereof;

(14) an aminopyrimidine derivative or a pharmacologically acceptable salt thereof according to (13), wherein in the general formula (I):

$R^6$ is a group of general formula (II):

-A-Y-B-Z (II)

wherein:
A is a single bond;

Y, B and Z have the same significance as defined in (13)];

(15) an aminopyrimidine derivative or a pharmacologically acceptable salt thereof according to (13), wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

-A-Y-B-Z (II)

wherein:
A is a single bond;
Y is an alkylene group which may be substituted;
B is an oxygen atom or a single bond; and
Z is an aryl group which may be substituted; provided that when A is a single bond and B is a single bond, Z is a substituted aryl group and the substituent is a group of formula:

-G-E

wherein G is an oxygen atom or an alkylene group which may be substituted and E is an aryl group which may be substituted;

(16) an aminopyrimidine derivative or a pharmacologically acceptable salt thereof according to (13), wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

-A-Y-B-Z (II)

wherein:
A is a single bond;
Y is an alkylene group which may be substituted;
B is a single bond; and
Z is a substituted aryl group and the substituent is a group of formula:

-G-E

wherein G is an oxygen atom or an alkylene group which may be substituted and E is an aryl group which may be substituted;

(17) an aminopyrimidine derivative or a pharmacologically acceptable salt thereof according to (13), wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

-A-Y-B-Z (II)

wherein:
A is a single bond;
Y is an alkylene group which may be substituted;
B is an oxygen atom; and
Z is an aryl group which may be substituted;

(18) An aminopyrimidine derivative or a pharmacologically acceptable salt thereof according to (13), wherein in the general formula (I):

$R^6$ is a group of general formula (II):

-A-Y-B-Z (II)

wherein:
A is a carbonyl group;
Y is an alkylene group which may be substituted;
B is an oxygen atom or a single bond; and
Z is an aryl group which may be substituted or a heterocyclic group which may be substituted;

(19) an aminopyrimidine derivative or a pharmacologically acceptable salt thereof according to (13), wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

-A-Y-B-Z (II)

wherein:
A is a carbonyl group;
Y is an alkylene group which may be substituted;
B is an oxygen atom or a single bond; and
Z is an aryl group which may be substituted;

(20) An aminopyrimidine derivative selected from the group consisting of the compounds below, or a pharmacologically acceptable salt thereof:

N-[2-(2,4-dimethylphenoxy)ethyl]-5,6-dimethyl-[1,2,4]triazolo[1,5-a]pyrimidin-7-amine;
N-{3-[4-(tert-butyl)phenoxy]propyl}-5,6-dimethyl-[1,2,4]triazolo[1,5-a]pyrimidin-7-amine;
5,6-dimethyl-N-{2-[4-(1-methyl-1-phenylethyl)phenoxy]-ethyl}[1,2,4]triazolo[1,5-a]pyrimidin-7-amine;
N-[2-{2,3-dimethyl-4-[4-(methylsulfanyl)phenoxy]-phenoxy}ethyl]-5,6-dimethyl[1,2,4]triazolo[1,5-a]pyrimidin-7-amine;
N-{2-[4-(2-chloro-4-fluorobenzyl)phenoxy]ethyl}-5,6-dimethyl[1,2,4]triazolo[1,5-a]pyrimidin-7-amine;
5,6-dimethyl-N-[2-(2-methyl-4-phenoxyphenoxy)ethyl]-[1,2,4]triazolo[1,5-a]pyrimidin-7-amine;
N-[2-(4-benzyl-2-ethylphenoxy)ethyl]-5,6-dimethyl-[1,2,4]triazolo[1,5-a]pyrimidin-7-amine;
N-[2-(2,4-dimethylphenoxy)ethyl]-5,6,7,8-tetrahydro-[1,2,4]triazolo[5,1-b]quinazolin-9-amine;
N-[2-([1,1'-biphenyl]-4-yloxy)ethyl]-5,6-dimethyl-[1,2,4]triazolo[1,5-a]pyrimidin-7-amine;
N-{2-[4-(2,4-dichlorobenzyl)phenoxy]ethyl}-5,6-dimethyl[1,2,4]triazolo[1,5-a]pyrimidin-7-amine;
N-{2-[4-(benzyloxy)phenoxy]ethyl}-5,6-dimethyl-[1,2,4]triazolo[1,5-a]pyrimidin-7-amine;
N-{2-[4-(3,4-dimethylbenzyl)phenoxy]ethyl}-5,6-dimethyl[1,2,4]triazolo[1,5-a]pyrimidin-7-amine;
5-methyl-N-[2-4(phenoxyphenoxy)ethyl][1,2,4]triazolo-[1,5-a]pyrimidin-7-amine;
N-{2-[4-(tert-butyl)phenoxy]ethyl}-5,6-dimethyl-[1,2,4]triazolo[1,5-a]pyrimidin-7-amine;
5,6-dimethyl-N-[2-(4-phenoxyphenoxy)ethyl][1,2,4]-triazolo[1,5-a]pyrimidin-7-amine;
N-[2-(4-benzylphenoxy)ethyl]-5,6-dimethyl-[1,2,4]-triazolo[1,5-a]pyrimidin-7-amine;
5,6-dimethyl-N-[2-(1-naphthyloxy)ethyl][1,2,4]triazolo-[1,5-a]pyrimidin-7-amine;
5,6-dimethyl-N-[2-(2-naphthyloxy)ethyl][1,2,4]triazolo-[1,5-a]pyrimidin-7-amine;
N-[3-(4-benzylphenoxy)propyl]-5,6-dimethyl[1,2,4]-triazolo[1,5-a]pyrimidin-7-amine;
N-[2-(2-bromo-4-phenoxyphenoxy)ethyl]-5,6-dimethyl-[1,2,4]triazolo[1,5-a]pyrimidin-7-amine;
N-[2-(2-chloro-4-phenoxyphenoxy)ethyl]-5,6-dimethyl-[1,2,4]triazolo[1,5-a]pyrimidin-7-amine;
N-[2-(4-benzyl-2-chlorophenoxy)ethyl]-5,6-dimethyl-[1,2,4]triazolo[1,5-a]pyrimidin-7-amine;
N-[2-(2,6-dimethyl-4-phenoxyphenoxy)ethyl]-5,6-dimethyl[1,2,4]triazolo[1,5-a]pyrimidin-7-amine;
N-(2-{2,6-dimethyl-4-[4-(methylsulfanyl)phenoxy]-phenoxy}ethyl)-5,6-dimethyl[1,2,4]triazolo[1,5-a]pyrimidin-7-amine;
N-{2-[4-(2,4-dimethylphenoxy)-2,6-dimethylphenoxy]-ethyl}-5,6-dimethyl[1,2,4]triazolo[1,5-a]pyrimidin-7-amine;
5,6-dimethyl-N-(2-{4-[4-(methylsulfanyl)phenoxy]-phenoxy}ethyl)[1,2,4]triazolo[1,5-a]pyrimidin-7-amine;
N-[2-(2-fluoro-4-phenoxyphenoxy)ethyl]-5,6-dimethyl-[1,2,4]triazolo[1,5-a]pyrimidin-7-amine;
N-[2-(2,3-dimethyl-4-phenoxyphenoxy)ethyl]-5,6-dimethyl[1,2,4]triazolo[1,5-a]pyrimidin-7-amine;
5,6-dimethyl-N-{2-[4-(phenylsulfanyl)phenoxy]ethyl}-[1,2,4]triazolo[1,5-a]pyrimidin-7-amine;
N-[2-(4-benzyl-2,3-dimethylphenoxy)ethyl]-5,6-dimethyl-[1,2,4]triazolo[1,5-a]pyrimidin-7-amine;
5,6-dimethyl-N-{2-[4-(4-methylbenzyl)phenoxy]ethyl}-[1,2,4]triazolo[1,5-a]pyrimidin-7-amine;
N-{2-[4-(2-fluorobenzyl)phenoxy]ethyl}-5,6-dimethyl-[1,2,4]triazolo[1,5-a]pyrimidin-7-amine;
5,6-dimethyl-N-(2-{4-[4-(methylsulfanyl)benzyl]-phenoxy}ethyl)[1,2,4]triazolo[1,5-a]pyrimidin-7-amine;
5,6-dimethyl-N-{2-[4-(1-phenylethyl)phenoxy]ethyl}-[1,2,4]triazolo[1,5-a]pyrimidin-7-amine;
N-[2-(4-benzyl-2-methylphenoxy)ethyl]-5,6-dimethyl-[1,2,4]triazolo[1,5-a]pyrimidin-7-amine;
N-[2-(4-benzylphenoxy)ethyl]-5-methyl[1,2,4]triazolo-[1,5-a]pyrimidin-7-amine;
N-[2-(4-benzyl-2-methylphenoxy)ethyl]-5-methyl[1,2,4]-triazolo[1,5-a]pyrimidin-7-amine;
N-[2-(2-methyl-4-phenoxyphenoxy)ethyl]-N-(5-methyl-[1,2,4]triazolo[1,5-a]pyrimidin-7-yl)amine;
N-[2-(4-benzyl-2-methylphenoxy)ethyl]-5-ethyl[1,2,4]-triazolo[1,5-a]pyrimidin-7-amine;
N-[2-(4-benzyl-2-ethylphenoxy)ethyl]-5-methyl[1,2,4]-triazolo[1,5-a]pyrimidin-7-amine;
N-[2-(4-benzyl-2,6-dimethylphenoxy)ethyl]-5,6-dimethyl-[1,2,4]triazolo[1,5-a]pyrimidin-7-amine;
N-[2-(4-benzyl-2,6-dimethylphenoxy)ethyl]-5-methyl-[1,2,4]triazolo[1,5-a]pyrimidin-7-amine;
N-[2-(4-benzyl-2-methylphenoxy)ethyl] [1,2,4]triazolo-[1,5-a]pyrimidin-7-amine;
5,6-dimethyl-N-{4-[4-(methylsulfanyl)phenoxy]benzyl}-[1,2,4]triazolo[1,5-a]pyrimidin-7-amine;
5,6-dimethyl-N-{4-[4-(methylsulfinyl)phenoxy]benzyl}-[1,2,4]triazolo[1,5-a]pyrimidin-7-amine;
5,6-dimethyl-N-{4-[4-(methylsulfonyl)phenoxy]benzyl}-[1,2,4]triazolo[1,5-a]pyrimidin-7-amine;
N-[2-(2,4-dimethylphenoxy)ethyl]-5,6-dimethylpyrazolo-[1/5-a]pyrimidin-7-amine;
N-{2-[4-(tert-butyl)phenoxy]ethyl}-5,6-dimethylpyrazolo[1,5-a]pyrimidin-7-amine;
N-[2-(4-benzyl-2-methylphenoxy)ethyl]-5,6-dimethylpyrazolo[1,5-a]pyrimidin-7-amine;
5,6-dimethyl-N-[2-(2-methyl-4-phenoxyphenoxy)ethyl]-pyrazolo[1,5-a]pyrimidin-7-amine;
N-[2-(4-benzyl-2,3-dimethylphenoxy)ethyl]-5,6-dimethylpyrazolo[1,5-a]pyrimidin-7-amine;
N-[2-(4-benzyl-2-methylphenoxy)ethyl]pyrazolo[1,5-a]pyrimidin-7-amine; and
N-[2-(4-benzylphenoxy)ethyl]-2-(tert-butyl)-5-methylpyrazolo[1,5-a]pyrimidin-7-amine;

(21) an aminopyrimidine derivative or a pharmacologically acceptable salt thereof according to any one of (13) through (20) for use as an active ingredient of a pharmaceutical composition;
(22) use of an aminopyrimidine derivative or a pharmacologically acceptable salt thereof according to any one of (1) through (20) in the production of a pharmaceutical composition for suppressing fat accumulation;
(23) a method for suppressing the accumulation of fat which comprises administering to a subject of interest an

effective dose of an aminopyrimidine derivative or a pharmacologically acceptable salt thereof according to any one of (1) through (20);

(24) a method for preparing a mature adipocyte population which comprises bringing a substance having prostaglandin $J_2$ activity and a differentiation inducing substance in contact with a confluent preadipocyte population, which is separated from animal adipose tissues and has a population doubling level of not greater than 4 after the separation, followed by incubation;

(25) a method for preparing a mature adipocyte population according to (24), wherein the animal is a mammal;

(26) a method for preparing a mature adipocyte population according to (24) or (25), wherein the animal is a rat;

(27) a method for preparing a mature adipocyte population according to any one of (24) to (26), wherein the adipose tissues are adipose tissues in the vicinity of mesenterium;

(28) a method for assaying a fat accumulation suppressing activity of a test substance which comprises culturing a mature adipocyte population prepared by the method according to any one of (24) to (27) in contact with a test substance or a control substance, measuring a fat content of the cell population cultured, and determining a level of fat accumulation suppression in the mature adipocyte population cultured in contact with the test substance, based on a difference between the fat content in the cell population cultured in contact with the test substance and the fat content in the cell population cultured in contact with the control substance;

(29) a method for assaying a fat accumulation suppressing activity of a test substance according to (28), wherein the cultured mature adipocyte population is stained with oil red O and the fat content in the cell population is measured based on the staining intensity;

(30) a method for screening a substance capable of suppressing the accumulation of fat, which comprises using the assay method according to (28) or (29) to determine a suppression level of fat accumulation in the mature adipocyte population cultured in contact with a test substance and selecting the test substance in terms of a degree of the suppression;

(31) a substance capable of suppressing the accumulation of fat which is selected by the screening method according to (30); and,

(32) a pharmaceutical composition for suppressing the accumulation of fat comprising as an active ingredient a substance capable of suppressing the accumulation of fat according to (31).

BEST MODE FOR CARRYING OUT THE INVENTION

Hereinafter the terms used throughout the specification are described below in detail.

The following description on the respective groups also applies, unless otherwise indicated, to such groups that are also substituents on each of other groups and other groups are substituted on the respective groups.

Examples of the alkyl group include C1-C12 alkyl groups such as methyl, ethyl, n-propyl, isopropyl, n-butyl, t-butyl, isobutyl, sec-butyl, n-pentyl, n-hexyl, 2-methylpentyl, n-heptyl, n-octyl, n-decyl, n-dodecyl, etc.

The substituent on the alkyl group which may be substituted may be one or more substituents. Examples of such substituents include a halogen atom, a C1-C12 alkoxy group, a hydroxy group, a mercapto group, an $-S(O)_n$ (C1-C12 alkyl group)(wherein n is 0, 1 or 2; hereinafter the same), a C3-C12 cycloalkyl group, an amino group which may be substituted, a heterocyclic group which may be substituted, etc.

Examples of the alkenyl group include C2-C12 alkenyl groups such as vinyl, 1-propenyl, allyl, isopropenyl, 1-butenyl, 1-pentenyl, 1-hexenyl, 1-octenyl, etc.

The alkenyl group which may be substituted may be substituted with one or more substituents. Examples of such substituents are a halogen atom, a C1-C12 alkoxy group, a hydroxy group, a mercapto group, an $S(O)_n$(C1-C12 alkyl group), an amino group which may be substituted, etc.

Examples of the aryl group are an aryl group having 10 or less carbon atoms such as phenyl, 1- or 2-naphthyl.

In the aryl group which may be substituted, the substituted phenyl and the substituted phenyloxy group may be substituted with one or more substituents. Examples of such substituents include: a halogen atom; a C1-C12 haloalkyl group; a C1-C12 alkyl group; a C2-C12 alkenyl group; a C1-C12 alkoxy group; a hydroxy group; a nitro group; a cyano group; a mercapto group; an $-S(O)_n$(C1-C12 alkyl group); a carboxy group; an ester group; an amino group which may be substituted; an amido group which may be substituted; a urea group which may be substituted; a sulfonamido group which may be substituted; a phenyl group which may be substituted with a halogen atom, a C1-C12 haloalkyl group, a C1-C12 alkyl group, a C2-C12 alkenyl group, a C1-C12 alkoxy group, a hydroxy group, a nitro group, a cyano group, a mercapto group, an $-S(O)_n$(C1-C12 alkyl group), a carboxy group or an ester group; an aralkyl group which may be substituted; a heterocyclic group which may be substituted; a phenyloxy group which may be substituted with a halogen atom, a C1-C12 haloalkyl group, a C1-C12 alkyl group, a C2-C12 alkenyl group, a C1-C12 alkoxy group, a hydroxy group, a nitro group, a cyano group, a mercapto group, an $-S(O)_n$(C1-C12 alkyl group), a carboxy group or an ester group; and the like.

In particular, in the general formula (I) wherein R6 is the general formula (II):

-A-Y-B-Z (II)

and Z is a substituted aryl group or a substituted heterocyclic group, preferred examples of substituents in the substituted aryl group or substituted heterocyclic group are a halogen atom; a C1-C12 haloalkyl group; a C1-C12 alkyl group; a C1-C12 alkoxy group and a group shown by formula:

-G-E

wherein G is an oxygen atom or an alkylene group which may be substituted and E is an aryl group which may be substituted or a heterocyclic group which may be substituted.

More preferred examples of the group shown by formula -G-E include:

(a) an aralkyl group which may be substituted; and
(b) a phenyloxy group which may be substituted with a halogen atom, a C1-C12 haloalkyl group, a C1-C12 alkyl group, a C2-C12 alkenyl group, a C1-C12 alkoxy group, a hydroxy group, a nitro group, a cyano group, a mercapto group, an -S(O)$_n$(C1-C12 alkyl group), a carboxy group or an ester group.

In the aralkyl group, examples of the aryl moiety are an aryl group having 10 carbon atoms or less, such as phenyl group, a 1- or 2-naphthyl group, etc.; and examples of the alkyl moiety are an alkyl group having 5 carbon atoms or less, such as a methyl group, an ethyl group, a propyl group, a butyl group, etc. Representative examples of the aralkyl group are a benzyl group, a 1- or 2-phenethyl group, etc.

The aralkyl group which may be substituted may be substituted with one or more substituents on the aryl moiety and/or on the alkyl moiety. Examples of such substituents are a halogen atom, a C1-C12 haloalkyl group, a C1-C12 alkyl group, a C2-C12 alkenyl group, a C1-C12 alkoxy group, a hydroxy group, a nitro group, a mercapto group, an -S(O)$_n$(C1-C12 alkyl group), a carboxy group, an ester group, an amino group which may be substituted, a phenyloxy group which may be substituted, and the like.

Examples of the heterocyclic group include a 5- or 6-membered heterocyclic group formed by 1 to 4 hetero atoms selected from nitrogen, oxygen and sulfur atoms and carbon atoms, such as a 2-pyridyl group, a 3-pyridyl group, a 4-pyridyl group, a 2-imidazolyl group, a pyrazinyl group, a 2-pyrimidinyl group, a 3-pyridazinyl group, a 3-oxadiazolyl group, a 2-thiazolyl group, a 3-isothiazolyl group, a 2-oxazolyl group, a 3-isoxazolyl group, a 2-furyl group, a 3-furyl group, a 2-thienyl group, a 3-thienyl group, a 2-quinolyl group, an 8-quinolyl group, a 2-quinazolinyl group, an 8-purinyl group, a 1-pyrrolyl group, a 1-pyrazolyl group, a 1-imidazolyl group, a 1,2,4-triazol-1-yl group, a tetrahydropyran-4-yl group, a tetrahydrofuran-3-yl group, a tetrahydrothiphen-3-yl group, a pyrrolidin-2-yl group, a pyrrolidin-3-yl group, a piperidin-2-yl group, a piperidin-3-yl group, a piperidin-4-yl group, a homopiperidin-2-yl group, a homopiperidin-3-yl group, a homopiperidin-4-yl group, a morpholin-2-yl group, etc.

In the heterocyclic group which may be substituted, the substituent are 1 or 2 selected independently from substituents such as a halogen atom, a C1-C12 alkyl group, a C2-C12 alkenyl group, a C1-C12 alkoxy group, a hydroxy group, a mercapto group, an -S(O)$_n$(C1-C12 alkyl group), a carboxy group, an ester group, an amino group which may be substituted.

The halo or halogen atom means chloro, bromo, fluoro or iodo.

Examples of the alkylene group are a C1 to C12 alkylene group such as a methylene group, an ethylene group, a trimethylene group, a tetramethylene group, a pentamethylene group, a hexamethylene group, a propylene group, an ethylethylene group, etc.

The alkylene group which may be substituted may be substituted with one or more substituents and examples of such substituents include a halogen atom, a C1-C12 alkoxy group, a hydroxy group, a mercapto group, an oxo group, a thioxo group, an -S(O)$_n$(C1-C12 alkyl group), an amino group which may be substituted, and the like. As the alkylene group substituted with such substituents, there are a hydroxymethylene, a 1-methoxyethylene, a 2-halobutylene, a 1-oxomethylene, etc.

As the acyl group, there are an alkanoyl group and an aroyl group. Examples of the alkanoyl group are groups substituted with carbonyl group at the terminus of the alkyl group exemplified above. Examples of the aroyl group are groups substituted with carbonyl group at the terminus of the aryl group exemplified above.

Examples of the alkoxy group include a C1-C12 alkoxy group such as a methoxy group, an ethoxy group, a n-propyloxy group, an isopropyloxy group, a n-butyloxy group, an isobutyloxy group, a sec-butyloxy group, a tert-butyloxy group, a n-pentyloxy group, an isopentyloxy group, a neopentyloxy group, a tert-pentyloxy group, a 1-ethylpropyloxy group, a n-hexyloxy group, an isohexyloxy group, a 2-ethylbutyloxy group, a 1-methylpentyloxy group, a 1-ethylbutyloxy group, a 3-methylpentyloxy group, a 1,3-dimethylbutyloxy group, and the like.

Examples of the cycloalkyl group include a C3-C12 cycloalkyl group such as a cyclopropyl group, a cyclobutyl group, a cyclopentyl group, a cyclohexyl group, a cycloheptyl group, a cyclooctyl group, a cyclodecyl group, a cyclododecyl group, etc.

Examples of the substituted amino group include a substituted amino group in which one or two hydrogens of the amino group are independently substituted with a C1-C12 alkyl group, a C2-C12 alkenyl group, a C1-C12 alkoxy group, a hydroxy group, and the like.

Examples of the haloalkyl group include a C1-C12 haloalkyl group such as a trifluoromethyl group, a trichloromethyl group, a tribromomethyl group, a difluoromethyl group, a monofluoromethyl group, a 1,1-difluoroethyl group, a 2,2,2-trifluoroethyl group, a pentafluoroethyl group, a 1,1-dichloro-2,2,2-trifluoroethyl group, a nonafluoro-n-butyl group, a nonafluoro-t-butyl group, etc.

The ester group means a carboxyl group which is esterified and includes, e.g., an alkoxycarbonyl group, an aryloxycarbonyl group, an aralkyloxycarbonyl group, etc. Specific examples include a methoxycarbonyl group, an ethoxycarbonyl group, a phenoxycarbonyl group, a benzyloxycarbonyl group, a 1- or 2-phenethyloxycarbonyl group, etc.

The amide group which may be substituted is a group shown by $-NR^7COR^8$, wherein $R^7$ is a hydrogen atom, a C1-C12 alkyl group, etc. and $R^8$ is a C1-C12 haloalkyl group, a C1-C12 alkyl group which may be substituted, a C2-C12 alkenyl group which may be substituted, a C3-C12 cycloalkyl group which may be substituted, a phenyl group which may be substituted, an aralkyl group which may be substituted, a heterocyclic group which may be substituted, etc.

The urea group which may be substituted is a group shown by $-NR^9CONR^{10}R^{11}$, wherein $R^9$ and $R^{10}$, which may be the same or different, represent a hydrogen atom, a C1-C12 alkyl group, etc. and $R^{11}$ is a C1-C12 haloalkyl group, a C1-C12 alkyl group which may be substituted, a C2-C12 alkenyl group which may be substituted, a C3-C12 cycloalkyl group which may be substituted, a phenyl group which may be substituted, an aralkyl group which may be substituted, a heterocyclic group which may be substituted, etc.

The sulfonamide group which may be substituted is a group shown by $-NR^{12}SO_2R^{13}$, wherein $R^{12}$ is a hydrogen atom, a C1-C12 alkyl group, etc. and $R^{13}$ is a C1-C12 haloalkyl group, a C1-C12 alkyl group which may be substituted, a C2-C12 alkenyl group which may be substituted, a C3-C12 cycloalkyl group which may be substituted, a phenyl group which may be substituted, an aralkyl group which may be substituted, a heterocyclic group which may be substituted, etc.

As described above, the pharmaceutical composition for suppressing the fat accumulation of the present invention comprises as an active ingredient pharmacologically acceptable salts of the compounds represented by formula (I) *supra.* The specific compounds of the present invention can react with a number of inorganic acids, organic acids or inorganic bases to form pharmaceutically acceptable salts. As acids normally employed to form acid addition salts, there are inorganic acids, e.g., hydrochloric acid, hydrobromic acid, sulfuric acid, phosphoric acid, etc. Examples of organic carboxylic acids are formic acid, acetic acid, fumaric acid, maleic acid, malic acid, tartaric acid, aspartic acid, glutamic acid, etc. Examples of the sulfonic acids are methanesulfonic acid, benzenesulfonic acid, p-toluenesulfonic acid, hydroxybenzenesulfonic acid, dihydroxybenzenesulfonic acid, etc. The base addition salts include those derived from inorganic bases such as hydroxides, carbonates, bicarbonates, etc. of ammonium, alkali metals, alkaline earth metals and the like. Examples of the bases useful for preparing the base addition salts include sodium hydroxide, potassium hydroxide, ammonium hydroxide, potassium carbonate, sodium carbonate, sodium bicarbonate, potassium bicarbonate, calcium hydroxide, calcium carbonate, etc. Potassium and sodium are particularly preferred.

Furthermore, the compounds *supra* or salts thereof which are active ingredients in the pharmaceutical composition of the present invention may be not only in the form of anhydrides but in the form of hydrates such as monohydrates or dihydrates, or solvates.

As the aminopyrimidine derivatives represented by the formula (I) *supra* or pharmacologically acceptable salts thereof, which are active ingredients of the pharmaceutical composition for suppressing the fat accumulation of the present invention, the aminopyrimidine derivatives shown in (2) through (11) *supra* or their pharmacologically acceptable salts are preferable. Specifically, the compound listed in (12) above is preferable.

Among the aminopyrimidine derivatives represented by the formula (I) above or pharmacologically acceptable salts thereof, the compounds shown in (13) above are novel and the compounds shown in (14) through (19) above are preferred novel compounds. Specifically, the compounds shown in (20) above are preferred novel compounds.

Process I:

In the compounds of the present invention represented by the general formula (I) above, the aminopyrimidine derivatives wherein $R^6$ is a C1 to C12 alkyl group which may be substituted, a C2-C12 alkenyl group which may be substituted, or a group of general formula (II):

$$-A-Y-B-Z \quad (II)$$

wherein:

A is a single bond;
Y is an alkylene group which may be substituted;
B is an oxygen atom or a single bond; and
Z is an aryl group which may be substituted or a heterocyclic group which may be substituted, can be produced by reacting halopyrimidine derivatives represented by general formula (III):

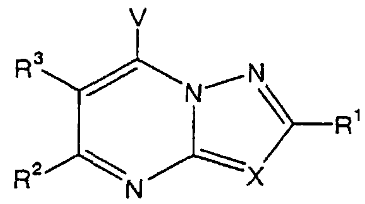

(III)

wherein $R^1$, $R^2$, $R^3$ and X have the same significance as defined above, and V is a halogen atom, with substituted alkylamine derivatives represented by general formula (IV):

$$NHR^5R^{6a} \quad (IV)$$

wherein $R^5$ has the same significance as defined above and $R^{6a}$ is a C1 to C12 alkyl group which may be substituted, a C2-C12 alkenyl group which may be substituted, or a group of general formula (II):

$$-A-Y-B-Z \quad (II)$$

wherein:
A is a single bond;
Y is an alkylene group which may be substituted;
B is an oxygen atom or a single bond; and
Z is an aryl group which may be substituted or a heterocyclic group which may be substituted, if necessary, in an appropriate organic solvent in the presence of an acid scavenger.

As the organic solvent used in the reaction, there are an organic solvent inert to the reaction, e.g., an aromatic hydrocarbon solvent such as toluene, benzene, chlorobenzene, xylene, etc.; a halogenated hydrocarbon solvent such as carbon tetrachloride, dichloromethane, 1,2-dichloroethane, etc.; a ketone solvent such as methyl ethyl ketone, methyl isobutyl ketone, etc.; an ethereal solvent such as tetrahydrofuran, 1,4-dioxane, etc. The reaction may also be carried out in the absence of any solvent.
Examples of the acid scavenger which can be used in the reaction are an organic base such as triethylamine, diisopropylethylamine, dimethylaminopyridine, pyridine, N,N-dimethylaniline, N,N-diethylaniline, etc.; an inorganic base such as sodium hydroxide, potassium hydroxide, sodium hydrogencarbonate, potassium carbonate, etc. The inorganic base is employed in a solid state, preferably powdery state, and suspended in a reaction solvent. An amount of the acid scavenger used is normally 1 to 2 equivalents, preferably 1.1 to 1.5 equivalents, based on 1 equivalent of the halopyrimidine derivative (III).
An amount of the substituted alkylamine (IV) used for the reaction is normally 1 to 3 equivalents, preferably 1.1 to 1.5 equivalents, based on 1 equivalent of the halopyrimidine derivative (III).
The reaction temperature is generally between room temperature and 150°C, preferably 50°C to 120°C, and the reaction time ranges generally from 30 minutes to 48 hours. The aminopyrimidine derivatives (I) produced in the reaction can be obtained, after completion of the reaction, by post-treatments including washing with water, extraction, drying, concentration, etc. If necessary, the compounds may also be subjected to operations such as column chromatography, recrystallization, etc.

As shown by Scheme 1 described below, the halopyrimidine derivatives (III) can be prepared by methods known to one skilled in the art, e.g., by the methods described in Japanese Patent KOKAI (Laid-Open) No. 62-67084, Indian Journal of Chemistry, Sec. B, vol. 27, pp. 825, 1988, etc.

Scheme 1

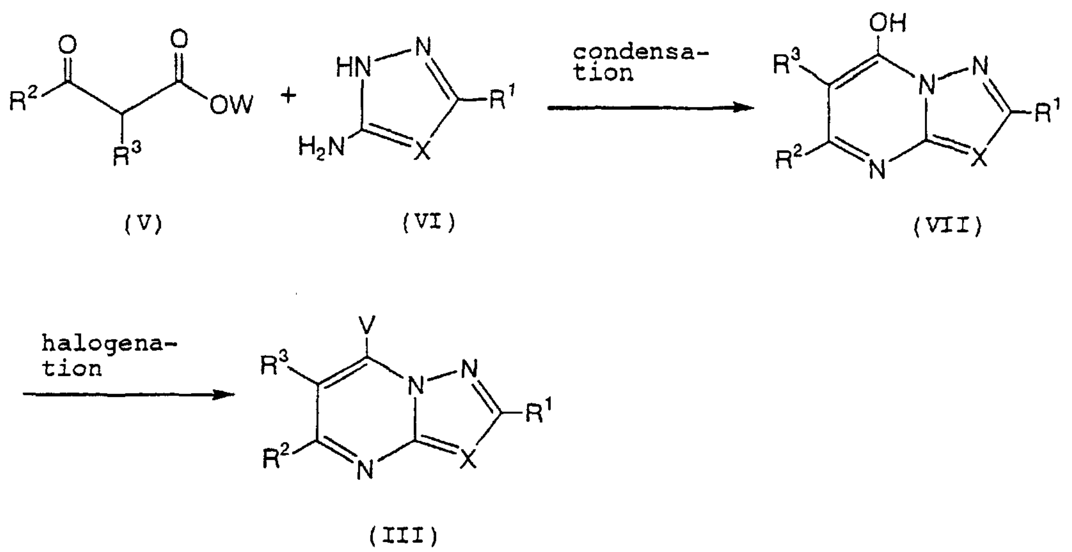


(V) (VI) (VII) (III)

wherein $R^1$, $R^2$, $R^3$ and X have the same significance as defined above, V represents a halogen atom and W represents a C1-C4 alkyl group.

That is, the halopyrimidine derivatives can be produced by condensing β-keto esters (V) with 3-amino-1,2,4-triazoles (VI, X=N) or 3-aminopyrazoles (VI, X= C-$R^4$, wherein $R^4$ has the same significance as defined above) followed by halogenation.

The reaction between compounds (V) and compounds (VI) is carried out in an appropriate inert solvent under temperature conditions ranging from room temperature to the boiling point of the solvent. Examples of the inert solvent used in this reaction include acetic acid, a lower alcohol solvent such as ethanol, methanol, isopropyl alcohol, etc.; an aromatic hydrocarbon solvent such as toluene, benzene, chlorobenzene, xylene, etc.; and an ethereal solvent such as tetrahydrofuran, 1,4-dioxane, etc.

Preferably, a ratio of the compounds (V) to the compounds (VI) used is generally in an almost equimolar amount. The reaction time is generally from 30 minutes to 24 hours, preferably about 2 hours to about 8 hours.

The compounds (III) can be prepared by reacting hydroxypyrimidine derivatives (VII) with an appropriate halogenating agent in the presence of an appropriate acid scavenger.

Examples of the acid scavenger which can be used in the reaction are organic basic substances such as triethylamine, diisopropylethylamine, dimethylaminopyridine, pyridine, N,N-dimethylaniline, N,N-diethylaniline, etc.; inorganic basic substances such as sodium hydroxide, potassium hydroxide, sodium hydrogencarbonate, potassium carbonate, etc. In the case of inorganic basic substances, the substances are employed after dispersing solid or preferably powdery substances in a reaction solvent. An amount of the acid scavenger used is normally 1 to 10 equivalents based on 1 equivalent of the hydroxypyrimidine derivatives (VII).

As the halogenating agent used in the reaction, there are, e.g., phosphorous oxychloride, phosphorous oxybromide, etc. Since the halogenating agent is also used as a solvent, it is unnecessary to use any other reaction solvent but an aromatic hydrocarbon solvent such as benzene, toluene, xylene, etc. may be employed.

The reaction temperature is generally between room temperature and 150°C, and the reaction time ranges generally from 30 minutes to 12 hours.

The substituted alkylamines (IV) are commercially available or can be prepared by subjecting cyanoalkyl derivatives (X)/(XIII) to catalytic hydrogenation or reduction with lithium aluminum hydride by methods known to one skilled in the art, for example, by the method shown in Scheme 2.

Scheme 2:

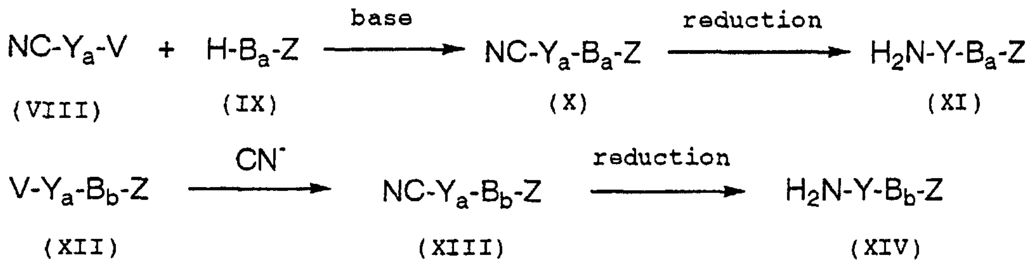


(VIII) (IX) (X) (XI)

(XII) (XIII) (XIV)

wherein V, Y and Z have the same significance as defined above, Ya represents an optionally substituted alkylene group which is shorter by one carbon at the cyano group side than Y, Ba is an oxygen atom, and Bb is a single bond.

Process II:

In the compounds of the present invention represented by the general formula (I) above, the aminopyrimidine derivatives wherein $R^6$ is an acyl group or a group of general formula (II):

$$-A-Y-B-Z \qquad (II)$$

wherein:

A is a carbonyl group;
Y is an alkylene group which may be substituted;
B is an oxygen atom or a single bond; and
Z is an aryl group which may be substituted or a heterocyclic group which may be substituted, can be produced by reacting aminopyrimidine derivatives represented by general formula (XV):

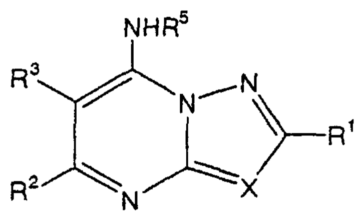

(XV)

wherein $R^1$, $R^2$, $R^3$ and $R^5$ have the same significance as defined above, with acid halides represented by general formula (XVI):

$$R^6V \qquad (XVI)$$

wherein $R^6$ and V have the same significance as defined above, in an appropriate organic solvent in the presence of an acid scavenger.

As the organic solvent used for the reaction, there are an organic solvent inert to the reaction, e.g., an aromatic hydrocarbon solvent such as toluene, benzene, chlorobenzene, xylene, etc.; a halogenated hydrocarbon solvent such as carbon tetrachloride, dichloromethane, 1,2-dichloroethane, etc.; a ketone solvent such as methyl ethyl ketone, methyl isobutyl ketone, etc.; an ethereal solvent such as diethyl ether, tetrahydrofuran, 1,4-dioxane, etc.

Examples of the acid scavenger which can be used in the reaction are a tertiary amine such as triethylamine, diisopropylethylamine, dimethylaminopyridine, pyridine, N,N-dimethylaniline, N,N-diethylaniline, etc.; an alkali metal

hydride such as sodium hydride, potassium hydride, etc.

Amounts of the acid halide (XVI) and the acid scavenger used in the reaction above are not particularly limited but the acid halide is normally used in approximately an equimolar to 2-fold molar equivalents and the acid scavenger preferably in an equimolar amount to an excess amount, based on the aminopyrimidine derivative (XV). The acid scavenger may also be used as a reaction solvent.

The reaction is carried out preferably under temperature conditions ranging from room temperature to a reflux temperature, and the reaction time is preferably from 30 minutes to 24 hours.

The aminopyrimidine derivatives (I) above may also be synthesized by condensation between carboxylic acid derivatives of general formula (XVII), instead of the acid halides (XVI):

$$R^6OH \qquad (XVII)$$

wherein $R^6$ has the same significance as defined above, and the aminopyrimidine derivatives (XV).

As the organic solvent used for the reaction, there are an organic solvent inert to the reaction, e.g., a halogenated hydrocarbon solvent such as carbon tetrachloride, dichloromethane, 1,2-dichloroethane, etc.; an ethereal solvent such as diethyl ether, tetrahydrofuran, 1,4-dioxane, etc.; N,N-dimethylformaide, and the like.

Examples of the condensing agent used for the reaction are condensing agents often used for peptide bond formation such as dicyclohexylcarbodiimide, diisopropylcarbodiimide, N-ethyl-N'-3-dimethylaminopropylcarbodiimide and hydrochlorides thereof, benzotriazol-1-yl-tris(dimethylamino)phosphonium hexafluorophosphide, diphenylphosphorylazide, etc., and carbonyl diimidazole, 2-ethoxy-1-ethoxycarbonyl-1,2-dihydroquinoline, triphenylphosphine-carbon tetrachloride, diethyl cyanophosphonate, diphenyl phosphoroazide, etc.

Also, for the purpose of accelerating the rate of condensation or controlling side reactions, there may be used additives such as N-hydroxysuccinimide, 1-hydroxybenzotriazole, 3,4-dihydro-3-hydroxy-4-oxo-1,2,3-benzotriazine, etc.

Amounts of the carboxylic acid (XVII) and the condensing agent used in the reaction above are not particularly limited but both are normally used in approximately equimolar to 2-fold molar equivalents, based on the aminopyrimidine derivative (XV). The additive is employed preferably in an equimolar to 2-fold molar amount based on the condensing agent.

The reaction is carried out preferably under temperature conditions ranging from room temperature to a reflux temperature, and the reaction time is preferably from about 30 minutes to about 24 hours.

The aminopyrimidine derivatives (XV) can be prepared according to Process I, using alkylamine derivatives of general formula (XVIII), instead of the alkylamine derivatives (IV):

$$R^5NH_2 \qquad (XVIII)$$

wherein $R^5$ has the same significance as defined above.

Also, the aminopyrimidine derivatives (XV) wherein $R^5$ is a hydrogen atom may also be prepared by the method as shown in Scheme 3, by modifying methods known to one skilled in the art, e.g., the method described in Japanese Patent KOKAI (Laid-Open) No. 8-310951.

Scheme 3:

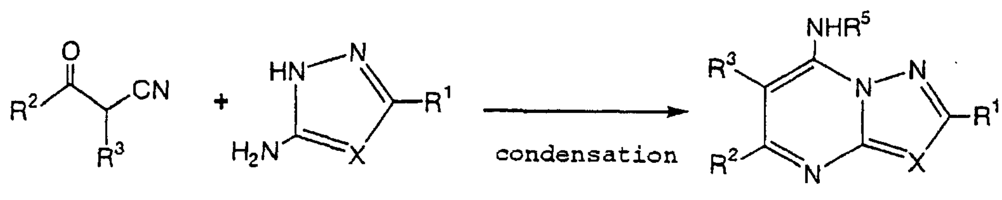


(XIX) (VI) (XV)

wherein $R^1$, $R^2$, $R^3$ and X have the same significance as defined above, and $R^5$ is a hydrogen atom.

That is, the reaction between the nitrile compounds (XIX) and the compounds (VI) is carried out under temperature conditions ranging from room temperature to the boiling point of the solvent in an appropriate inert solvent.

Examples of the inert solvent used in this reaction include acetic acid, a lower alcohol solvent such as ethanol, methanol, isopropyl alcohol, etc.; an aromatic hydrocarbon solvent such as toluene, benzene, chlorobenzene, xylene, etc.; and an ethereal solvent such as tetrahydrofuran, 1,4-dioxane, etc.

Preferably, a ratio of the compounds (XIX) to the compounds (VI) used is generally in an almost equimolar amount. The reaction temperature is preferably in the range from room temperature to a reflux temperature and the reaction time is generally from about 30 minutes to about 24 hours.

The compounds represented by the general formula (I) and salts thereof exhibit the activity of suppressing the accumulation of fat in fat cells and can thus be utilized for suppressing an increase in adipose tissues and for the prevention and treatment of diseases accompanied by increased adipose tissues. More specifically, these compounds are useful as drugs for the prevention and treatment of obesity accompanied by increased body fat or increased adipose tissues in the abdominal cavity, impairment in glucose tolerance, diabetes mellitus, hyperglycemia, hyperlipemia, etc. The compounds are thus expected to be effective for the prevention and treatment of hypertension, coronary arterial diseases, obstructive arterial sclerosis and the like.

When the compounds represented by the general formula (I) and salts thereof are utilized as drugs, they may be administered orally or parenterally. That is, the compounds may be administered orally in a conventional form such as a tablet, capsule, syrup, suspension or the like, or parenterally by injecting the compounds in the form of a solution, emulsion, suspension or the like. In addition, the compounds may also be administered rectally in the form of a suppository. The above suitable forms of dosage can be prepared by incorporating the active compound into an acceptable conventional carrier, excipient, binder, stabilizer, or the like. In addition, when the active compound is used in the injection forms, an acceptable buffer, solubilizing aid, isotonic agent or the like may be added thereto. The dosage and frequency depend upon the symptom, age and body weight as well as the dosage form, but the dosage is typically in the range of approximately 1 to 2000 mg per day, preferably about 5 to 1000 mg per day, when orally given to adult, and in the range of approximately 0.1 to 500 mg per day when injected; in the case of injection, the dose may be given in single administration or divided into a multiplicity of administrations (preferably 2 to 4 times).

The compounds having the aforesaid activity of suppressing the accumulation of fat in fat cells can be screened by testing an effect of a test substance on the accumulation of fat in mature fat cells.

The mature adipocyte population that can be used for the above test can be prepared by the method of the present invention for preparing a mature adipocyte population, which comprises bringing a substance having prostaglandin $J_2$ activity and a differentiation inducing substance in contact with a confluent preadipocyte population, which is separated from animal adipose tissues and has a population doubling level of not greater than 4 after the separation, followed by incubation.

In the method of the present invention for preparing the mature adipocyte population, a preadipocyte population is first separated and prepared from animal adipose tissues. Herein the preadipocyte population is used to mean a population of cells abundant in preadipocytes. Examples of adipose tissues that can be used to prepare the cell population are adipose tissues present in the subcutaneous abdominal region, femoral region, gluteal region, pectoral region, etc. and in the abdominal cavity in the vicinity of the mesenterium, kidney, epididymis, etc. of mammals including rat, mouse, hamster, monkey, human, etc. Specifically, the cell population can be prepared, e.g., by finely mincing the adipose tissues isolated from rat, digesting the minced tissues with an enzyme such as collagenase, etc.; fractionating the resulting cell suspension by centrifugal separation, etc. according to a method modified from, e.g., the method by Shillabeer G. et al., International Journal of Obesity, 20, S77-S83, etc.; and then recovering a preadipocyte-rich fraction.

Next, the thus obtained preadipocyte population is cultured and when a population doubling level of the cell population after separation from the tissues is 4 or less, a differentiation induction treatment is performed. The preadipocyte population which is subjected to the differentiation induction treatment is previously cultured to be confluent. The differentiation induction is effected by a method which comprises incorporating a differentiation inducing substance, e.g., insulin, dexamethasone, 3-isobutyl-1-methylxanthine, etc. into a medium to bring the differentiation inducing substance in contact with the preadipocyte population, more specifically by a modification of the method described in Wu Z. et al., Genes & Dev., 9, 2350-2363, etc. In the method of the present invention for preparing the mature adipocyte population, a substance having prostaglandin $J_2$ activity is added to a medium, together with the differentiation inducing substance to bring the same in contact with the preadipocyte population described above. Herein, the substance having prostaglandin $J_2$ activity refers to a substance that can differentiate the preadipocyte population into a cell population containing mature adipocytes when it is brought in contact with the preadipocyte population along with the differentiation inducing substance in such a rate as high as the case when the preadipocyte population is brought into contact with the differentiation inducing substance and prostaglandin $J_2$. Examples of the substance having prostaglandin $J_2$ activity include prostaglandin $J_2$, 15-deoxy-$\Delta^{12,14}$ -prostaglandin $J_2$, etc. The concentration of the substance having prostaglandin $J_2$ activity added to a medium is normally approximately 5 to 25 μM as a final concentration when the active substance is prostaglandin $J_2$, and normally approximately 1 to 10 μM as a final concentration when the active substance is 15-deoxy-$\Delta^{12,14}$-prostaglandin $J_2$. As described above, by bringing the substance having prostaglandin $J_2$ activity and the differentiation inducing substance in contact with the confluent preadipocyte population separated from

animal adipose tissues and having a population doubling level of not greater than 4, the cell population abundant in mature fat cells, namely, mature adipocyte population can be prepared. The method of the present invention for preparing the mature adipocyte population is preferable to known methods, in the point that the cell population containing mature fat cells in a higher proportion can be obtained, than by the known methods. In particular, the method of the present invention can be preferably used to prepare the mature adipocyte population derived from adipose tissues in the vicinity of the mesenterium.

For assaying the fat accumulation suppressing activity of a test substance using the thus prepared mature adipocyte population by the method of the present invention for preparing the mature adipocyte population *supra*, for example, a test substance or a control substance (e.g., a control solvent consisting of a solvent alone with no test substance) is added to a medium of the cell population followed by incubation to bring the cell population in contact with the substance for a given period of time, and a fat content in the cell population is measured to determine a level of the fat accumulation suppression in the mature adipocyte population cultured in contact with the test substance, based on a difference between the fat content in the cell population cultured in contact with the test substance and the fat content in the cell population cultured in contact with the control substance. As the test substance, not only a chemically synthesized compound but a naturally occurring substance, a protein or a peptide may be used. A suitable timing bringing the test substance in contact with the adipocyte population is generally from the moment when the substance having prostaglandin $J_2$ activity and the differentiation inducing substance are brought in contact with the adipocyte population according to the method of the present invention for preparing the mature adipocyte population to 5 days thereafter. A period of time for the contact is usually not shorter than an hour.

To determine the fat content in an adipocyte population, for example, a method which involves microscopically observing an adipocyte population thereby to visually judge the amount of fat drops caused by the accumulation of fat in cytoplasm of the respective cells, a method which involves degrading fat in the cells with an enzyme and quantifying the thus released glycerol to assay the fat content, and the like may be used. Another method involves staining an adipocyte population with Oil Red O, lysing cells in the cell population and quantifying the amount of Oil Red O in the lysate with a spectrophotometer, etc. may also be used. Thus, the fat content in the cell population is measured. The amount of glycerol produced by enzymatic degradation of fat in the cells can be assayed using kits commercially available, e.g., Determiner TG-S555 (manufactured by Kyowa Medex Co., Ltd.). Staining of adipocyte population with Oil Red O can be effected in a conventional manner described in, e.g., Novikoff, A. B. et al., J. Cell Biol., 87, 180-196, etc. Oil Red O is normally provided for use by dissolving the same in an aqueous solution of a hydrophilic solvent such as triethyl phosphate, etc. After staining, a surplus of the dye is washed off with the solvent diluted with water and a cell lysis agent is added to lyse the cells. The cell lysis agent may be an alkali solution, a surfactant, or a mixture of the two. The cell lysis agent is used generally in an amount of 50 to 200 μl per well of a 96-well plate. After the lysate is recovered, absorbance is measured and the amount of Oil Red O in the lysate is converted into the amount of fat. The absorbance measured is generally read at 450 to 550 nm. The measurement by staining with Oil Red O is preferable, since it gives a higher sensitivity compared to the visual observation of fat drops or the quantification of glycerol described above and also enables to assay in a simple manner in a short time, and is thus applicable to a system for assaying the fat accumulation controlling activity of many test substances, e.g., to a high through-put screening system, etc.

The activity of a test substance for suppressing the accumulation of fat can be determined by measuring the fat content of a mature adipocyte population cultured in contact with the test substance as described above, comparing the same with, e.g., the fat content in a mature adipocyte population cultured in the absence of test substance, e.g., adding a control solvent into the medium, and determining to what degree the fat accumulation in the mature adipocyte population is suppressed by contact between the cell population and the test substance. In a specific embodiment, when the fat content in a mature adipocyte population cultured in contact with a test substance is less than the fat content in a mature adipocyte population cultured in the absence of test substance, e.g., adding a control solvent into the medium, it is judged that the fat accumulation in the mature adipocyte population is suppressed by the contact with the test substance. In this case, the test substance is found to be capable of suppressing the accumulation of fat. Moreover, the level of the suppressing activity of the test substance can be determined. As described above, by selecting a test substance that gives a less fat content in a mature adipocyte population cultured in contact with the test substance than the fat content in the mature adipocyte population cultured in contact with a control substance, the substance having the fat accumulation suppressing activity can be screened. Furthermore, the substance having the fat accumulation suppressing activity can be selected, based on a degree of the fat accumulation suppression by contact with a test substance toward a mature adipocyte population. That is, by this screening method, substances having the fat accumulation suppressing activity can be selected from compounds chemically synthesized, naturally occurring substances, proteins, peptides, etc. These substances selected are effective for the prevention and treatment of various disorders such as obesity accompanied by the increase of adipose tissues, diabetes mellitus, hyperlipemia, and the like.

Hereinafter the present invention will be described in more detail with reference to EXAMPLES and TEST

EXAMPLES but is not deemed to be limited thereto.

EXAMPLE 1

After 0.51 g (2.79 mmols) of 7-chloro-5,6-dimethyl[1,2,4]triazolo[1,5-a]pyrimidine, 0.59 g (3.58 mmols) of 2-(2,4-dimethylphenoxy)ethylamine and 2.5 ml of toluene were charged, the temperature was elevated to 100°C. While maintaining the reaction temperature at 100°C, 0.36 g (3.56 mmols) of triethylamine was added to the mixture. Subsequently, the mixture was stirred at the same temperature for 3 hours. After completion of the reaction, the reaction mass was poured onto 10 ml of 5% aqueous hydrochloric acid solution followed by extraction with 10 ml of toluene. The extracted organic phase was further washed with 10 ml of water. After drying over magnesium sulfate, the organic phase was filtered and the filtrate was concentrated. The resulting residue was purified by silica gel column chromatography to give 0.21 g of N-[2-(2,4-dimethylphenoxy)ethyl]-5,6-dimethyl[1,2,4]triazolo[1,5-a]pyrimidin-7-amine.
Melting point: 139.2°C

In a manner similar to EXAMPLE 1, compounds shown in EXAMPLES 2 through 38 were obtained.

EXAMPLE 2

N-{3-[4-(tert-Butyl)phenoxy]propyl}-5,6-dimethyl-[1,2,4]triazolo[1,5-a]pyrimidin-7-amine
Melting point: 144.4°C

EXAMPLE 3

5,6-Dimethyl-N-{2-[4-(1-methyl-1-phenylethyl)phenoxy]-ethyl}[1,2,4]triazolo[1,5-a]pyrimidin-7-amine
Melting point: 118.1°C

EXAMPLE 4

N-(2-{2,3-Dimethyl-4-[4-(methylsulfanyl)phenoxy]-phenoxy}ethyl)-5,6-dimethyl[1,2,4]triazolo[1,5-a]pyrimidin-7-amine
Melting point: 131.5°C

EXAMPLE 5

N-{2-[4-(2-Chloro-4-fluorobenzyl)phenoxy]ethyl}-5,6-dimethyl[1,2,4]triazolo[1,5-a]pyrimidin-7-amine
Melting point: 159.8°C

EXAMPLE 6

5,6-Dimethyl-N-[2-(2-methyl-4-phenoxyphenoxy)ethyl]-[1,2,4]triazolo[1,5-a]pyrimidin-7-amine
Melting point: 131.0°C

EXAMPLE 7

N-[2-(4-Benzyl-2-ethylphenoxy)ethyl]-5,6-dimethyl[1,2,4]triazolo[1,5-a]pyrimidin-7-amine
Melting point: 146.5°C

EXAMPLE 8

N-[2-(2,4-Dimethylphenoxy)ethyl]-5,6,7,8-tetrahydro-[1,2,4]triazolo[5,1-b]quinazolin-9-amine
Melting point: 151.4°C

EXAMPLE 9

N-[2-([1,1'-Biphenyl]-4-yloxy)ethyl]-5,6-dimethyl-[1,2,4]triazolo[1,5-a]pyrimidin-7-amine
Melting point: 158.6°C

EXAMPLE 10

N-{2-[4-(2,4-Dichlorobenzyl)phenoxy]ethyl}-5,6-dimethyl[1,2,4]triazolo[1,5-a]pyrimidin-7-amine
Melting point: 164.4°C

EXAMPLE 11

N-{2-[4-(Benzyloxy)phenoxy]ethyl}-5,6-dimethyl[1,2,4]-triazolo[1,5-a]pyrimidin-7-amine
Melting point: 126.8°C

EXAMPLE 12

N-{2-[4-(tert-Butyl)phenoxy]ethyl}-5,6-dimethyl[1,2,4]-triazolo[1,5-a]pyrimidin-7-amine
Melting point: 146.3°C

EXAMPLE 13

5,6-Dimethyl-N-[2-(4-phenoxyphenoxy)ethyl][1,2,4]-triazolo[1,5-a]pyrimidin-7-amine
Melting point: 78.7°C

EXAMPLE 14

N-[2-(4-Benzylphenoxy)ethyl]-5,6-dimethyl[1,2,4]-triazolo[1,5-a]pyrimidin-7-amine
Melting point: 118.2°C

EXAMPLE 15

5,6-Dimethyl-N-[2-(1-naphthyloxy)ethyl][1,2,4]triazolo-[1,5-a]pyrimidin-7-amine
Melting point: 148.7°C

EXAMPLE 16

5,6-Dimethyl-N-[2-(2-naphthyloxy)ethyl][1,2,4]triazolo-[1,5-a]pyrimidin-7-amine
Melting point: 154.5°C

EXAMPLE 17

N-[2-(2,6-Dimethyl-4-phenoxyphenoxy)ethyl]-5,6-dimethyl[1,2,4]triazolo[1,5-a]pyrimidin-7-amine
Melting point: 137.8°C

EXAMPLE 18

N-(2-{2,6-Dimethyl-4-[4-(methylsulfanyl)phenoxy]-phenoxy}ethyl)-5,6-dimethyl[1,2,4]triazolo[1,5-a]pyrimidin-7-amine
Melting point: 120.6°C

EXAMPLE 19

N-{2-[4-(2,4-Dimethylphenoxy)-2,6-dimethylphenoxy]-ethyl}-5,6-dimethyl[1,2,4]triazolo[1,5-a]pyrimidin-7-amine
Melting point: 123.8°C

EXAMPLE 20

5,6-Dimethyl-N-(2-{4-[4-(methylsulfanyl)phenoxy]-phenoxy}ethyl)[1,2,4]triazolo[1,5-a]pyrimidin-7-amine
Melting point: 76.5°C

EXAMPLE 21

N-[2-(2-Fluoro-4-phenoxyphenoxy)ethyl]-5,6-dimethyl-[1,2,4]triazolo[1,5-a]pyrimidin-7-amine
Melting point: 100.5°C

EXAMPLE 22

N-[2-(2,3-Dimethyl-4-phenoxyphenoxy)ethyl]-5,6-dimethyl[1,2,4]triazolo[1,5-a]pyrimidin-7-amine
Melting point: 124.8°C

EXAMPLE 23

N-[2-(4-Benzyl-2,3-dimethylphenoxy)ethyl]-5,6-dimethyl[1,2,4]triazolo[1,5-a]pyrimidin-7-amine
Melting point: 143.0°C

EXAMPLE 24

[1,2,4]triazolo[1,5-a]pyrimidin-7-amine
Melting point: 158.7°C

EXAMPLE 25

N-{2-[4-(2-Fluorobenzyl)phenoxy]ethyl}-5,6-dimethyl-[1,2,4]triazolo[1,5-a]pyrimidin-7-amine
Melting point: 109.6°C

EXAMPLE 26

5,6-Dimethyl-N-(2-{4-[4-(methylsulfanyl)benzyl]-phenoxy}ethyl)[1,2,4]triazolo[1,5-a]pyrimidin-7-amine
Melting point: 91.5°C

EXAMPLE 27

5,6-Dimethyl-N-{2-[4-(1-phenylethyl)phenoxy]ethyl}-[1,2,4]triazolo[1,5-a]pyrimidin-7-amine
Melting point: 61.5°C

EXAMPLE 28

N-[2-(4-Benzyl-2-methylphenoxy)ethyl]-5,6-dimethyl-[1,2,4]triazolo[1,5-a]pyrimidin-7-amine
Melting point: 127.6°C

EXAMPLE 29

N-[2-(4-Benzylphenoxy)ethyl]-5-methyl[1,2,4]triazolo-[1,5-a]pyrimidin-7-amine
Melting point: 165.0°C

EXAMPLE 30

N-[2-(4-Benzyl-2-methylphenoxy)ethyl]-5-methyl-[1,2,4]triazolo[1,5-a]pyrimidin-7-amine
Melting point: 185.1°C

EXAMPLE 31

N-[2-(2-Methyl-4-phenoxyphenoxy)ethyl]-5-methyl-[1,2,4]triazolo[1,5-a]pyrimidin-7-amine
Melting point: 157.4°C

EXAMPLE 32

N-[2-(4-Benzyl-2-ethylphenoxy)ethyl]-5-methyl[1,2,4]-triazolo[1,5-a]pyrimidin-7-amine

Melting point: 148.1°C

EXAMPLE 33

N-[2-(4-Benzyl-2,6-dimethylphenoxy)ethyl]-5,6-dimethyl-[1,2,4]triazolo[1,5-a]pyrimidin-7-amine
Melting point: 126.5°C

EXAMPLE 34

N-[2-(4-Benzyl-2,6-dimethylphenoxy)ethyl]-5-methyl-[1,2,4]triazolo[1,5-a]pyrimidin-7-amine
$N_D$ (22.5°C): 1.6062

EXAMPLE 35

N-[2-(4-Benzyl-2-methylphenoxy)ethyl][1,2,4]triazolo-[1,5-a]pyrimidin-7-amine
Melting point: 140.3°C

EXAMPLE 36

5,6-Dimethyl-N-{4-[4-(methylsulfanyl)phenoxy]benzyl}-[1,2,4]triazolo[1,5-a]pyrimidin-7-amine
Melting point: 143°C

EXAMPLE 37

5,6-Dimethyl-N-{4-[4-(methylsulfinyl)phenoxy]benzyl}-[1,2,4]triazolo[1,5-a]pyrimidin-7-amine
Melting point: 167°C

EXAMPLE 38

5,6-Dimethyl-N-{4-[4-(methylsulfonyl)phenoxy]benzyl}-[1,2,4]triazolo[1,5-a]pyrimidin-7-amine
Melting point: 178°C

EXAMPLE 39

After 0.51 g (3.00 mmols) of 7-chloro-5,6-dimethylpyrazolo[1,5-a]pyrimidine, 0.59 g (3.58 mmols) of 2-(2,4-dimethylphenoxy)ethylamine and 2.5 ml of toluene were charged, the temperature was elevated to 100°C. While maintaining the reaction temperature at 100°C, 0.36 g (3.56 mmols) of triethylamine was added to the mixture. Subsequently, the mixture was stirred at the same temperature for 3 hours. After completion of the reaction, the reaction mass was poured onto 10 ml of 5% aqueous hydrochloric acid solution followed by extraction with 10 ml of toluene. The extracted organic phase was further washed with 10 ml of water. After drying over magnesium sulfate, the organic phase was filtered and the filtrate was concentrated. The resulting residue was purified by silica gel column chromatography to give 0.13 g of N-[2-(2,4-dimethylphenoxy)ethyl]-5,6-dimethylpyrazolo[1,5-a]pyrimidin-7-amine.
Melting point: 98.5°C

In a manner similar to EXAMPLE 39, compounds shown in EXAMPLES 40 through 43 were obtained.

EXAMPLE 40

N-{2-[4-(tert-Butyl)phenoxy]ethyl}-5,6-dimethylpyrazolo[1,5-a]pyrimidin-7-amine
Melting point: 165.4°C

EXAMPLE 41

N-[2-(4-Benzyl-2-methylphenoxy)ethyl]-5,6-dimethylpyrazolo[1,5-a]pyrimidin-7-amine
Melting point: 124.7°C

EXAMPLE 42

5,6-Dimethyl-N-[2-(2-methyl-4-phenoxyphenoxy)ethyl]-pyrazolo[1,5-a]pyrimidin-7-amine

Melting point: 122.8°C

EXAMPLE 43

N-[2-(4-Benzylphenoxy)ethyl]-2-(tert-butyl)-5-methylpyrazolo[1,5-a]pyrimidin-7-amine
Melting point: 105.9°C

TEST EXAMPLE 1

(1) Isolation of adipose tissues

Thirty-two Wistar male rats of 14 week age (Nippon SLC Co., Ltd.) were sacrificed and adipose tissues attached to the mesenterium (hereinafter referred to as mesenteric adipose tissues) were all isolated by laparotomy. Also, subcutaneous adipose tissues located from the ventral region to the femoral region (hereinafter referred to as the subcutaneous abdominal adipose tissues) were isolated only from one side per rat. These tissues collected were immersed, respectively, in a phosphate buffer (0.20 g/L KCl, 0.20 g/L $KH_2PO_4$, 8.00 g/L NaCl, 2.16 g/L $Na_2HPO_4 \cdot 7H_2O$, 100 units/ml penicillin (manufactured by GIBCO), 100 μg/ml streptomycin (manufactured by GIBCO), 250 ng/ml amphotericin (manufactured by GIBCO)), followed by washing at room temperature.

(2) Preparation and cultivation of the cells from test tissues

After washing, the mesenteric adipose tissues and the subcutaneous abdominal adipose tissues were subjected to the following treatment, respectively. That is, these tissues were finely minced with scissors in about 300 ml of Dulbecco-modified Eagle's medium (containing 4.5 g/L D-glucose and 584 mg/L L-glutamine, manufactured by GIBCO) supplemented with collagenase (Type II or VIII, manufactured by Sigma), penicillin (manufactured by GIBCO), streptomycin (manufactured by GIBCO) and amphotericin (manufactured by GIBCO), in final concentrations of 1 mg/ml, 100 units/ml, 100 μg/ml and 250 ng/ml, respectively. The tissues thus minced into about 5 mm dice were shaken at 37°C for 60 minutes (ca. 170 rpm) and filtered through a nylon mesh (805 [a mesh size of 250 μm], manufactured by Sanshin Kogyo Co., Ltd.) to recover the filtrate (cell suspension). After the filtrate was centrifuged at room temperature for 5 minutes at 1800 rpm, the liquid layer was gently removed by decantation to obtain precipitates. The precipitates were suspended in 50 ml of Dulbecco-modified Eagle's medium (containing 4.5 g/L D-glucose and 584 mg/L L-glutamine, manufactured by GIBCO) supplemented with fetal bovine serum (hereinafter abbreviated as FBS) (manufactured by GIBCO), ascorbic acid (manufactured by Wako Pure Chemical Industries), penicillin, streptomycin (manufactured by GIBCO) and amphotericin (manufactured by GIBCO), in final concentrations of 10%, 200 μM, 100 units/ml, 100 μg/ml and 250 ng/ml, respectively. The suspension was filtered through a nylon mesh (420S [a mesh size of 25 μm], manufactured by Sanshin Kogyo Co., Ltd.). After the filtrate was recovered and centrifuged at room temperature for 5 minutes at 1800 rpm, the liquid layer was gently removed by decantation. The precipitates were resuspended in 50 ml of the medium *supra* (hereinafter referred to as FBS-containing medium). With the suspension, the operation cycle of centrifugation, removal of the liquid layer and suspension in the FBS-containing medium was further repeated twice in a manner described above, provided that the final precipitates were suspended in 120 ml of the FBS-containing medium. The cell suspension was thus prepared. The cell suspension was dispensed by 30 ml each in a cell culture flask (T150 for adhesion cells, manufactured by Iwaki Glass Co., Ltd.) followed by culturing at 37°C in the presence of 5% CO2. Two or three hours after the initiation of incubation, the medium was removed and the flask wall was washed with 15 ml of the phosphate buffer *supra.* The washing liquid was removed. After the washing was again performed, the phosphate buffer was removed and 30 ml of FBS-containing medium was charged in the flask followed by incubating at 37°C in the presence of 5% CO2. On one day after the initiation of incubation, the medium was withdrawn and the flask wall was washed once with 15 ml of phosphate buffer. Thereafter a trypsinethylenedinitrotetraacetic acid (hereinafter abbreviated as EDTA) solution (0.05% trypsin and 0.53 mM EDTA·4Na, manufactured by GIBCO) was poured till the cells were barely covered, which was allowed to stand at 37°C for 5 minutes. The FBS-containing medium was added thereto in a 10-fold volume of the trypsin-EDTA solution to give a cell suspension.

(3) Test A on the fat accumulation suppressing activity

Using the cells derived from mesenteric adipose tissues, the fat accumulation suppressing activity of compounds was tested. The cells in the suspension prepared from the mesenteric adipose tissues in (2) *supra* were counted with a hemocytometer and diluted to $1.4 \times 10^5$ cells/ml by adding the FBS-containing medium. An aliquot of 100 μl each/well of the dispersion was dispensed in a 96-well plate (for culturing adhesion cells, manufactured by Iwaki Glass Co., Ltd.) followed by culturing at 37°C in the presence of 5% $CO_2$. Two or three days later, the medium was removed

from each well of the 96-well plate and 100 μl of the FBS-containing medium supplemented with 10 μg/ml insulin (manufactured by Sigma), 0.25 μM dexamethasone (Wako Pure Chemical Industries), 0.5 mM 3-isobutyl-1-methylxanthine (manufactured by Sigma) and 5 μM 15-deoxy-$\Delta^{12,14}$-prostaglandin $J_2$ (manufactured by Cayman) was added to each well followed by culturing at 37°C for 2 days in the presence of 5% $CO_2$. Subsequently, the medium in each well was withdrawn and 100 μl each of a FBS-containing medium supplemented with 10 μg/ml insulin and 5 μM 15-deoxy-$\Delta^{12,14}$-prostaglandin $J_2$ was added to each well. After culturing at 37°C for further 2 days in the presence of 5% $CO_2$, the medium in each well was withdrawn and 100 μl of the FBS-containing medium supplemented with 10 μg/ml insulin, 5 μM 15-deoxy-$\Delta^{12,14}$-prostaglandin $J_2$, 50 μM of a test compound and 0.5% dimethylsulfoxide (hereinafter abbreviated as DMSO)[Wako Pure Chemical Industries] was added to each well followed by culturing in the same manner. As for the group containing no test compound, 100 μl of the FBS-containing medium supplemented with 10 μg/ml insulin, 5 μM 15-deoxy-$\Delta^{12,14}$-prostaglandin $J_2$ and 0.5% DMSO was added to each well followed by culturing in the same manner as described above. After culturing for 2 days, the fat in the cells was stained with Oil Red O [Sudan II, Wako Pure Chemical Industries] and the fat content was measured by colorimetry. That is, first, 30 μl of a 0.075% Oil Red O staining liquid/60% triethyl phosphate solution was added directly to each well in which the cell culture medium was charged. After allowing to stand at room temperature for 30 minutes, the medium containing Oil Red O staining liquid was removed and 100 μl of 20% triethyl phosphate aqueous solution was added to each well. Following the addition, 20% triethyl phosphate aqueous solution was removed from each well and fresh 100 μl of the aqueous solution was added to each well. After repeating the procedure *supra,* 100 μl of solution to lysis the cell (2% SDS, 0.2N NaOH) was added to each well. After keeping at 37°C for at least 3 hours, absorbance was measured at a wavelength of 490 nm (hereinafter referred to as measured value 1), using a Plate Reader (Vmax, manufactured by Beckman). With regard to the group added with no test compound, the lipid in the cells was stained and absorbance was measured in the same way (hereinafter referred to as measured value 2). From these measurements, a fat accumulation suppressing rate by the test compound was calculated according to the following equation.

$$\text{Fat accumulation suppressing rate (\%)} = \{(\text{measured value 2} - \text{measured value 1})/\text{measured value 2}\} \times 100$$

The fat accumulation suppressing rates by test compounds were as follows: 51% in N-[2-(2,4-dimethyl-phenoxy)ethyl]-5,6-dimethyl[1,2,4]triazolo-[1,5-a]pyrimidin-7-amine; 83% in 5,6-dimethyl-N-{2-[4-(1-methyl-1-phenylethyl)phenoxy]ethyl}[1,2,4]-triazolo[1,5-a]pyrimidin-7-amine; 45% in N-(2-{2,3-dimethyl-4-[4-(methylsulfanyl)phenoxy]phenoxy}ethyl)-5,6-dimethyl[1,2,4]triazolo[1,5-a]pyrimidin-7-amine; 80% in 5,6-dimethyl-N-[2-(2-methyl-4-phenoxyphenoxy)-ethyl][1,2,4]triazolo[1,5-a]pyrimidin-7-amine; 72% in N-[2-(4-benzyl-2-ethylphenoxy)ethyl]-5,6-dimethyl-[1,2,4]triazolo[1,5-a]pyrimidin-7-amine; 76% in N-[2-(2,4-dimethylphenoxy)ethyl]-5,6-dimethylpyrazolo-[1,5-a]pyrimidin-7-amine; 52% in N-[2-(tert-butyl)pyrazolo-[1,5-a]pyrimidin-7-yl]-2,6-dimethoxybenzamide; and 44% in 2-(tert-butyl)-5-methyl-N-phenethylpyrazolo[1,5-a]pyrimidin-7-amine.

(4) Test B on the fat accumulation suppressing activity

Using the cells derived from subcutaneous abdominal adipose tissues, the fat accumulation suppressing activity by compounds was tested. The cells in the suspension prepared from the subcutaneous abdominal adipose tissues in (2) *supra* were counted with a hemocytometer and diluted to $1.4 \times 10^5$ cells/ml by adding the FBS-containing medium to the suspension. Using the dilution, cell culture was performed and each test compound was added to the cell by the same procedure as in (3). The lipid in the cells to which the test compound was added and the lipid in the cells to which no test compound was added were stained with Oil Red O [Sudan II, Wako Pure Chemical Industries] and their absorbance was measured (hereinafter the measurements for the cells added with the test compound and for the cells added with no test compound are referred to as measured values 3 and 4, respectively). From these measured values, a fat accumulation suppressing rate by the test compound was calculated according to the following equation.

$$\text{Fat accumulation suppressing rate (\%)} = \{(\text{measured value 4} - \text{measured value 3})/\text{measured value 4}\} \times 100$$

The fat accumulation suppressing rates by test compounds were as follows: 77% in 5,6-dimethyl-N-{2-[4-(1-methyl-1-phenylethyl)phenoxy]ethyl}-[1,2,4]triazolo[1,5-a]pyrimidin-7-amine; 75% in 5,6-dimethyl-N-[2-(2-methyl-4-phenoxyphenoxy)ethyl][1,2,4]-triazolo[1,5-a]pyrimidin-7-amine; 81% in N-[2-(4-benzyl-2-ethylphenoxy)ethyl]-5,6-dimethyl[1,2,4]-triazolo[1,5-a]pyrimidin-7-amine; 49% in N-[2-(2,4-dimethylphenoxy)ethyl]-5,6-dimethylpyrazolo[1,5-a]pyrimidin-7-amine; and 35% in 2-(tert-butyl)-5-methyl-N-phenethylpyrazolo[1,5-a]pyrimidin-7-amine.

TEST EXAMPLE 2

(1) Preparation of adipose tissue slice

In a manner similar to TEST EXAMPLE 1, the mesenteric adipose tissues and the subcutaneous abdominal adipose tissues were isolated from Wistar male rats of 14 week age and subjected to the following treatment, respectively. That is, the isolated tissues were first washed with 2 ml of Dulbecco-modified Eagle's medium (containing 4.5 g/L D-glucose and 584 mg/L L-glutamine, manufactured by GIBCO) followed by finely mincing in the medium with scissors in about 1 mm dice.

(2) Test C on the fat accumulation suppressing activity

Using the mesenteric adipose tissues or subcutaneous abdominal adipose tissue prepared in (1), the fat accumulation suppressing activity by compounds was tested. That is, first, 500 μl each/well of Dulbecco-modified Eagle's medium-low content glucose (containing 1.0 g/L D-glucose and 584 mg/L L-glutamine, manufactured by GIBCO) was dispensed in a 48-well plate (for culturing adhesion cells, manufactured by Sumitomo Bakelite Co., Ltd.) and a solution of a test compound dissolved in DMSO was further added to the well so as the test compound and DMSO to show final concentrations of 50 μM and 0.5%, respectively. In each well, 50 to 100 mg of the adipose tissue slice *supra* was charged followed by culturing at 37°C for 30 minutes in the presence of 5% CO2. In the group added with no test compound, DMSO alone was charged in a final concentration of 0.5%, instead of the DMSO solution of test compound described above, the adipose tissue slice was added to the well as described above, followed by incubation in the same way. Thirty minutes after the initiation of incubation, 15 μl of a radioisotope-labeled glucose solution (D-[U-$^{14}$C] glucose, 7.4 MBq/ml, manufactured by Amersham) was added to each well followed by culturing at 37°C for 7 hours in the presence of 5% $CO_2$. After that, the tissue slice in each well was transferred into 750 μl of heptane/isopropanol (heptane : isopropanol = 3:2), which was allowed to stand at room temperature for about 15 hours. Then, the tissue slice was withdrawn from the solution described above, and heptane and isopropanol were evaporated off. A 6 μl aliquot of the resulting residue was dissolved in 120 μl of chloroform/methanol (chloroform : methanol = 2:1). An 6 μl aliquot of the solution was spotted on a plate for thin layer chromatography (K5 silica gel 150 angstrom, manufactured by Whatman Co., hereinafter referred to as TLC plate). A developing solvent of hexane : ethyl ether : acetic acid (75:25:1) was charged in a closed vessel. After developing the TLC plate, the TLC plate was dried at room temperature and exposed to an imaging plate (BAS3-2040, manufactured by Fuji Photo Film Co., Ltd.) for 4 to 5 hours. The imaging plate after the exposure was analyzed with an image analyzer (BAS2000 Bioimage Analyzer, manufactured by Fuji Photo Film Co., Ltd.) to measure the radioactivity of the part corresponding to the developed position of triglycerides on the TLC plate (hereinafter referred to as measured value 5). The group added with no test compound was treated in the same manner, including thin layer chromatography, and the radioactivity of the part corresponding to the developed position of triglycerides on the TLC plate was measured (hereinafter referred to as measured value 6). From these measured values, a rate of suppressing the accumulation of fat (triglycerides) by the test compound was calculated according to the following equation.

$$\text{Fat accumulation suppressing rate (\%)} = \{(\text{measured value 6} - \text{measured value 5})/\text{measured value 6}\} \times 100$$

In the test using the mesenteric adipose tissues, the fat accumulation suppressing rates by test compounds were as follows: 51% in 5,6-dimethyl-N-{2-[4-(1-methyl-1-phenylethyl)phenoxy]ethyl}[1,2,4]-triazolo[1,5-a]pyrimidin-7-amine; 61% in 5,6-dimethyl-N-[2-(2-methyl-4-phenoxyphenoxy)ethyl][1,2,4]triazolo-[1,5-a]pyrimidin-7-amine; and 81% in N-[2-(4-benzyl-2-ethylphenoxy)ethyl]-5,6-dimethyl[1,2,4]triazolo[1,5-a]pyrimidin-7-amine. In the test using the subcutaneous abdominal adipose tissues, the fat accumulation suppressing rates by test compounds were as follows: 71% in 5,6-dimethyl-N-{2-[4-(1-methyl-1-phenylethyl)phenoxy]ethyl}[1,2,4]triazolo[1,5-a]pyrimidin-7-amine; 81% in 5,6-dimethyl-N-[2-(2-methyl-4-phenoxyphenoxy)ethyl][1,2,4]triazolo[1,5-a]pyrimidin-7-amine; and 80% in N-[2-(4-benzyl-2-ethylphenoxy)ethyl]-5,6-dimethyl[1,2,4]triazolo[1,5-a]pyrimidin-7-amine.

INDUSTRIAL APPLICABILITY

According to the present invention, the fat accumulation suppressor comprising the aminopyrimidine derivatives and salts thereof as active ingredients can be provided. In addition, use of the fat accumulation suppressor of the present invention is useful for suppressing the accumulation of fat on adipose tissues, thereby various disorders such as obesity accompanied by increased body fat or increased adipose tissues in the abdominal cavity, diabetes mellitus, hyperlipemia, etc. can be prevented and treated.

## Claims

**1.** A pharmaceutical composition for suppressing the accumulation of fat comprising as an active ingredient an aminopyrimidine derivative represented by general formula (I):

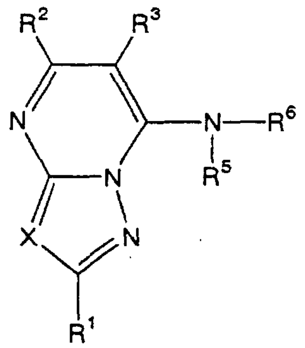

(I)

wherein:

$R^1$ is a hydrogen atom, an alkyl group which may be substituted, an alkenyl group which may be substituted, an aryl group which may be substituted, an aralkyl group which may be substituted, or a heterocyclic group which may be substituted;
$R^2$ and $R^3$, which may be the same or different, represent a hydrogen atom, a halogen atom, an alkyl group which may be substituted, an alkenyl group which may be substituted, an aryl group which may be substituted, an aralkyl group which may be substituted, or a heterocyclic group which may be substituted; or $R^2$ and $R^3$ may be combined together to form a C3 to C10 alkylene group which may be substituted;
X is a nitrogen atom or a group of C-$R^4$ wherein $R^4$ is a hydrogen atom, a halogen atom, an alkyl group which may be substituted, an alkenyl group which may be substituted, an aryl group which may be substituted, or an aralkyl group which may be substituted;
$R^5$ is a hydrogen atom, an alkyl group which may be substituted, or an alkenyl group which may be substituted;
$R^6$ is a C1 to C12 alkyl group which may be substituted, a C2-C12 alkenyl group which may be substituted, an acyl group, or a group of general formula (II):

-A-Y-B-Z (II)

wherein:
A is a carbonyl group or a single bond;
Y is an alkylene group which may be substituted;
B is an oxygen atom or a single bond; and
Z is an aryl group which may be substituted or a heterocyclic group which may be substituted; or a pharmacologically acceptable salt thereof.

**2.** A pharmaceutical composition for suppressing the accumulation of fat according to claim 1, wherein in the general formula (I):

$R^6$ is a C1 to C12 alkyl group which may be substituted, a C2-C12 alkenyl group which may be substituted,

or a group of general formula (II):

$$-A-Y-B-Z \quad (II)$$

wherein:
A is a single bond;
Y is an alkylene group which may be substituted;
B is an oxygen atom or a single bond; and
Z is an aryl group which may be substituted or a heterocyclic group which may be substituted.

**3.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a single bond;
Y is an alkylene group which may be substituted;
B is an oxygen atom; and
Z is an aryl group which may be substituted or a heterocyclic group which may be substituted.

**4.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a single bond;
Y is an alkylene group which may be substituted;
B is a single bond; and
Z is an aryl group which may be substituted or a heterocyclic group which may be substituted.

**5.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is an oxygen atom; and
Z is an aryl group which may be substituted or a heterocyclic group which may be substituted.

**6.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

-A-Y-B-Z (II)

wherein:
A is a single bond;
Y is an alkylene group which may be substituted;
B is a single bond; and
Z is an aryl group which may be substituted or a heterocyclic group which may be substituted.

**7.** A pharmaceutical composition for suppressing the accumulation of fat according to claim 1, wherein in the general formula (I):

$R^6$ is a C1 to C12 alkyl group which may be substituted, an C2 to C12 alkenyl group which may be substituted, or a group of general formula (II):

-A-Y-B-Z (II)

wherein:
A is a carbonyl group;
Y is an alkylene group which may be substituted;
B is an oxygen atom or a single bond; and
Z is an aryl group which may be substituted or a heterocyclic group which may be substituted.

**8.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

-A-Y-B-Z (II)

wherein:
A is a carbonyl group;

Y is an alkylene group which may be substituted;
B is an oxygen atom; and
Z is an aryl group which may be substituted or a heterocyclic group which may be substituted.

**9.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a carbonyl group;
Y is an alkylene group which may be substituted;
B is a single bond; and
Z is an aryl group which may be substituted or a heterocyclic group which may be substituted.

**10.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a carbonyl group;
Y is an alkylene group which may be substituted;
B is an oxygen atom; and
Z is an aryl group which may be substituted or a heterocyclic group which may be substituted.

**11.** A pharmaceutical composition for suppressing the accumulation of fat according to claim 1, wherein in the general formula (I):

$R^1$ is a hydrogen atom, an alkyl group which may be substituted, or an alkenyl group which may be substituted;
$R^2$ and $R^3$, which may be the same or different, represent a hydrogen atom, a halogen atom, or an alkyl group which may be substituted;
X is a nitrogen atom or a group of C-$R^4$ wherein $R^4$ is a hydrogen atom, a halogen atom, or an alkyl group which may be substituted;
$R^5$ is a hydrogen atom; and
$R^6$ is a group of general formula (II):

$$-A-Y-B-Z \quad (II)$$

wherein:
A is a carbonyl group;

Y is an alkylene group which may be substituted;
B is a single bond; and
Z is an aryl group which may be substituted or a heterocyclic group which may be substituted.

**12.** A pharmaceutical composition for suppressing the accumulation of fat according to claim 1, comprising an aminopyrimidine derivative selected from the group consisting of the compounds below, or a pharmacologically acceptable salt thereof:

N-[2-(2,4-dimethylphenoxy)ethyl]-5,6-dimethyl[1,2,4]-triazolo[1,5-a]pyrimidin-7-amine;
N-{3-[4-(tert-butyl)phenoxy]propyl}-5,6-dimethyl-[1,2,4]triazolo[1,5-a]pyrimidin-7-amine;
5,6-dimethyl-N-{2-[4-(1-methyl-1-phenylethyl)phenoxy]-ethyl}[1,2,4]triazolo[1,5-a]pyrimidin-7-amine;
N-[2-{2,3-dimethyl-4-[4-(methylsulfanyl)phenoxy]-phenoxy}ethyl]-5,6-dimethyl[1,2,4]triazolo[1,5-a]-pyrimidin-7-amine;
N-{2-[4-(2-chloro-4-fluorobenzyl)phenoxy]ethyl}-5,6-dimethyl[1,2,4]triazolo[1,5-a]pyrimidin-7-amine;
5,6-dimethyl-N-[2-(2-methyl-4-phenoxyphenoxy)ethyl]-[1,2,4]triazolo[1,5-a]pyrimidin-7-amine;
N-[2-(4-benzyl-2-ethylphenoxy)ethyl]-5,6-dimethyl-[1,2,4]triazolo[1,5-a]pyrimidin-7-amine;
N-[2-(2,4-dimethylphenoxy)ethyl]-5,6,7,8-tetrahydro-[1,2,4]triazolo[5,1-b]quinazolin-9-amine;
N-[2-([1,1'-biphenyl]-4-yloxy)ethyl]-5,6-dimethyl-[1,2,4]triazolo[1,5-a]pyrimidin-7-amine;
N-{2-[4-(2,4-dichlorobenzyl)phenoxy]ethyl}-5,6-dimethyl[1,2,4]triazolo[1,5-a]pyrimidin-7-amine;
N-{2-[4-(benzyloxy)phenoxy]ethyl}-5,6-dimethyl[1,2,4]-triazolo[1,5-a]pyrimidin-7-amine;
N-{2-[4-(3,4-dimethylbenzyl)phenoxy]ethyl}-5,6-dimethyl[1,2,4]triazolo[1,5-a]pyrimidin-7-amine;
5-methyl-N-[2-4(phenoxyphenoxy)ethyl][1,2,4]triazolo-[1,5-a]pyrimidin-7-amine;
N-{2-[4-(tert-butyl)phenoxy]ethyl}-5,6-dimethyl-[1,2,4]triazolo[1,5-a]pyrimidin-7-amine;
5,6-dimethyl-N-[2-(4-phenoxyphenoxy)ethyl]-[1,2,4]-triazolo[1,5-a]pyrimidin-7-amine;
N-[2-(4-benzylphenoxy)ethyl]-5,6-dimethyl[1,2,4]-triazolo[1,5-a]pyrimidin-7-amine;
5,6-dimethyl-N-[2-(1-naphthyloxy)ethyl] [1,2,4]triazolo-[1,5-a]pyrimidin-7-amine;
5,6-dimethyl-N-[2-(2-naphthyloxy)ethyl] [1,2,4]triazolo-[1,5-a]pyrimidin-7-amine;
N-[3-(4-benzylphenoxy)propyl]-5,6-dimethyl[1,2,4]-triazolo[1,5-a]pyrimidin-7-amine;
N-[2-(2-bromo-4-phenoxyphenoxy)ethyl]-5,6-dimethyl-[1,2,4]triazolo[1,5-a]pyrimidin-7-amine;
N-[2-(2-chloro-4-phenoxyphenoxy)ethyl]-5,6-dimethyl-[1,2,4]triazolo[1,5-a]pyrimidin-7-amine;
N-[2-(4-benzyl-2-chlorophenoxy)ethyl]-5,6-dimethyl-[1,2,4]triazolo[1,5-a]pyrimidin-7-amine;
N-[2-(2,6-dimethyl-4-phenoxyphenoxy)ethyl]-5,6-dimethyl[1,2,4]triazolo[1,5-a]pyrimidin-7-amine;
N-(2-{2,6-dimethyl-4-[4-(methylsulfanyl)phenoxy]-phenoxy}ethyl)-5,6-dimethyl[1,2,4]triazolo[1,5-a]-pyrimidin-7-amine;
N-{2-[4-(2,4-dimethylphenoxy)-2,6-dimethylphenoxy]-ethyl}-5,6-dimethyl[1,2,4]triazolo[1,5-a]pyrimidin-7-amine;
5,6-dimethyl-N-(2-{4-[4-(methylsulfanyl)phenoxy]-phenoxy}ethyl)[1,2,4]triazolo[1,5-a]pyrimidin-7-amine;
N-[2-(2-fluoro-4-phenoxyphenoxy)ethyl]-5,6-dimethyl-[1,2,4]triazolo[1,5-a]pyrimidin-7-amine;
N-[2-(2,3-dimethyl-4-phenoxyphenoxy)ethyl]-5,6-dimethyl[1,2,4]triazolo[1,5-a]pyrimidin-7-amine;
5,6-dimethyl-N-{2-[4-(phenylsulfanyl)phenoxy]ethyl}-[1,2,4]triazolo[1,5-a]pyrimidin-7-amine;
N-[2-(4-benzyl-2,3-dimethylphenoxy)ethyl]-5,6-dimethyl-[1,2,4]triazolo[1,5-a]pyrimidin-7-amine;
5,6-dimethyl-N-{2-[4-(4-methylbenzyl)phenoxy]ethyl}-[1,2,4]triazolo[1,5-a]pyrimidin-7-amine;
N-{2-[4-(2-fluorobenzyl)phenoxy]ethyl}-5,6-dimethyl-[1,2,4]triazolo[1,5-a]pyrimidin-7-amine;
5,6-dimethyl-N-(2-{4-[4-(methylsulfanyl)benzyl]-phenoxy}ethyl)[1,2,4]triazolo[1,5-a]pyrimidin-7-amine;
5,6-dimethyl-N-{2-[4-(1-phenylethyl)phenoxy]ethyl}-[1,2,4]triazolo[1,5-a]pyrimidin-7-amine;
N-[2-(4-benzyl-2-methylphenoxy)ethyl]-5,6-dimethyl-[1,2,4]triazolo[1,5-a]pyrimidin-7-amine;
N-[2-(4-benzylphenoxy)ethyl]-5-methyl[1,2,4]triazolo-[1,5-a]pyrimidin-7-amine;
N-[2-(4-benzyl-2-methylphenoxy)ethyl]-5-methyl[1,2,4]-triazolo[1,5-a]pyrimidin-7-amine;
N-[2-(2-methyl-4-phenoxyphenoxy)ethyl]-N-(5-methyl-[1,2,4]triazolo[1,5-a]pyrimidin-7-yl)amine;
N-[2-(4-benzyl-2-methylphenoxy)ethyl]-5-ethyl[1,2,4]-triazolo[1,5-a]pyrimidin-7-amine;
N-[2-(4-benzyl-2-ethylphenoxy)ethyl]-5-methyl[1,2,4]-triazolo[1,5-a]pyrimidin-7-amine;
N-[2-(4-benzyl-2,6-dimethylphenoxy)ethyl]-5,6-dimethyl-[1,2,4]triazolo[1,5-a]pyrimidin-7-amine;
N-[2-(4-benzyl-2,6-dimethylphenoxy)ethyl]-5-methyl-[1,2,4]triazolo[1,5-a]pyrimidin-7-amine;
N-[2-(4-benzyl-2-methylphenoxy)ethyl][1,2,4]triazolo-[1,5-a]pyrimidin-7-amine;
5,6-dimethyl-N-(3-phenoxyphenethyl)[1,2,4]triazolo-[1,5-a]pyrimidin-7-amine;
2,5-dimethyl-N-(3-methylphenyl)[1,2,4]triazolo[1,5-a]-pyrimidin-7-amine;
2,5-dimethyl-N-(3-methylbenzyl)[1,2,4]triazolo[1,5-a]-pyrimidin-7-amine;
N-[1-(1-benzothiophen-2-yl)ethyl]-5,6-dimethyl-[1,2,4]triazolo[1,5-a]pyrimidin-7-amine;

5,6-dimethyl-N-{4-[4-(methylsulfanyl)phenoxy]benzyl}-[1,2,4]triazolo[1,5-a]pyrimidin-7-amine;
5,6-dimethyl-N-{4-[4-(methylsulfinyl)phenoxy]benzyl}-[1,2,4]triazolo[1,5-a]pyrimidin-7-amine;
5,6-dimethyl-N-{4-[4-(methylsulfonyl)phenoxy]benzyl}-[1,2,4]triazolo[1,5-a]pyrimidin-7-amine;
N-[2-(2,4-dimethylphenoxy)ethyl]-5,6-dimethylpyrazolo-[1,5-a]pyrimidin-7-amine;
N-{2-[4-(tert-butyl)phenoxy]ethyl}-5,6-dimethylpyrazolo[1,5-a]pyrimidin-7-amine;
N-[2-(4-benzyl-2-methylphenoxy)ethyl]-5,6-dimethylpyrazolo[1,5-a]pyrimidin-7-amine;
5,6-dimethyl-N-[2-(2-methyl-4-phenoxyphenoxy)ethyl]-pyrazolo[1,5-a]pyrimidin-7-amine;
N-[2-(4-benzyl-2,3-dimethylphenoxy)ethyl]-5,6-dimethylpyrazolo[1,5-a]pyrimidin-7-amine;
N-[2-(4-benzyl-2-methylphenoxy)ethyl]pyrazolo[1,5-a]-pyrimidin-7-amine;
N-[2-(4-benzylphenoxy)ethyl]-2-(tert-butyl)-5-methylpyrazolo[1,5-a]pyrimidin-7-amine;
2-(tert-butyl)-N-(2,3-dimethoxybenzyl)-5-methylpyrazolo[1,5-a]pyrimidin-7-amine;
3-hydroxy-N-(2-methylpyrazolo[1,5-a]pyrimidin-7-yl)benzamide;
N-[2-(tert-butyl)pyrazolo[1,5-a]pyrimidin-7-yl]-2,6-dimethoxybenzamide;
2-(tert-butyl)-N-(2-methoxyphenethyl)-5-methylpyrazolo-[1,5-a]pyrimidin-7-amine;
2-(tert-butyl)-N-(3-methoxyphenethyl)-5-methylpyrazolo-[1,5-a]pyrimidin-7-amine;
2-(tert-butyl)-5-methyl-N-phenethylpyrazolo[1,5-a]-pyrimidin-7-amine;
2-(tert-butyl)-5-methyl-N-(3-phenylpropyl)pyrazolo-[1,5-a]pyrimidin-7-amine;
N-[2-(tert-butyl)-5-methylpyrazolo[1,5-a]pyrimidin-7-yl]-N-[4-(3,5-dimethoxyphenoxy)benzyl]amine;
N-[2-(tert-butyl)-5-methylpyrazolo[1,5-a]pyrimidin-7-yl]-N-[4-(3,5-dichlorophenoxy)benzyl]amine;
2-(tert-butyl)-N-(2-methoxybenzyl)-5-methylpyrazolo-[1,5-a]pyrimidin-7-amine;
2-(tert-butyl)-N-(4-methoxyphenethyl)-5-methylpyrazolo-[1,5-a]pyrimidin-7-amine;
2-(tert-butyl)-5-methyl-N-(4-pyridylmethyl)pyrazolo-[1,5-a]pyrimidin-7-amine;
2-(tert-butyl)-5-methyl-N-(4-phenylbutyl)pyrazolo-[1,5-a]pyrimidin-7-amine;
2-(tert-butyl)-5-methyl-N-(2-pyridylmethyl)pyrazolo-[1,5-a]pyrimidin-7-amine; and
2-(tert-butyl)-5-methyl-N-(3-pyridylmethyl)pyrazolo-[1,5-a]pyrimidin-7-amine.

**13.** An aminopyrimidine derivative represented by general formula (I):

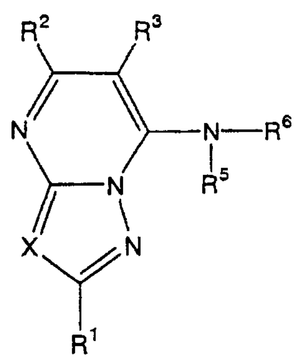

(I)

wherein:

$R^1$ is a hydrogen atom, an alkyl group which may be substituted, an alkenyl group which may be substituted, an aryl group which may be substituted, an aralkyl group which may be substituted, or a heterocyclic group which may be substituted;

$R^2$ and $R^3$, which may be the same or different, represent a hydrogen atom, a halogen atom, an alkyl group which may be substituted, an alkenyl group which may be substituted, an aryl group which may be substituted, an aralkyl group which may be substituted, or a heterocyclic group which may be substituted; or $R^2$ and $R^3$ may be combined together to form a C3 to C10 alkylene group which may be substituted;

X is a nitrogen atom or a group of C-$R^4$ wherein $R^4$ is a hydrogen atom, a halogen atom, an alkyl group which may be substituted, an alkenyl group which may be substituted, an aryl group which may be substituted, or an aralkyl group which may be substituted;

$R^5$ is a hydrogen atom, an alkyl group which may be substituted, or an alkenyl group which may be substituted;

$R^6$ is a group of general formula (II):

-A-Y-B-Z (II)

wherein:

A is a carbonyl group or a single bond;

Y is an alkylene group which may be substituted;

B is an oxygen atom or a single bond; and

Z is an aryl group which may be substituted or a heterocyclic group which may be substituted; provided that when A is a single bond and B is a single bond, Z is a substituted aryl group or a substituted heterocyclic group and the substituent for these groups of Z is a group of formula:

-G-E

wherein G is an oxygen atom or an alkylene group which may be substituted and E is an aryl group which may be substituted or a heterocyclic group which may be substituted;

provided that when $R^1$, $R^3$, $R^4$ and $R^5$ are hydrogen atoms, $R^2$ is a n-butyl group and A is a carbonyl group, Z is a heterocyclic group which may be substituted; or a pharmacologically acceptable salt thereof.

**14.** An aminopyrimidine derivative or a pharmacologically acceptable salt thereof according to claim 13, wherein in the general formula (I):

$R^6$ is a group of general formula (II):

-A-Y-B-Z (II)

wherein:

A is a single bond;

Y, B and Z have the same significance as defined in claim 13.

**15.** An aminopyrimidine derivative or a pharmacologically acceptable salt thereof according to claim 13, wherein in the general formula (I):

$R^1$ is a hydrogen atom or an alkyl group which may be substituted;

$R^2$ and $R^3$, which may be the same or different, represent a hydrogen atom or an alkyl group which may be substituted, or $R^2$ and $R^3$ may be combined together to form a C3 to C10 alkylene group which may be substituted;

X is a nitrogen atom or a group of C-H;

$R^5$ is a hydrogen atom; and

$R^6$ is a group of general formula (II):

-A-Y-B-Z (II)

wherein:

A is a single bond;

Y is an alkylene group which may be substituted;

B is an oxygen atom or a single bond; and

Z is an aryl group which may be substituted; provided that when A is a single bond and B is a single bond, Z is a substituted aryl group and the substituent is a group of formula:

-G-E

wherein G is an oxygen atom or an alkylene group which may be substituted and E is an aryl group which may be substituted.

**16.** An aminopyrimidine derivative or a pharmacologically acceptable salt thereof according to claim 13, wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

$$-A-Y-B-Z \qquad (II)$$

wherein:
A is a single bond;
Y is an alkylene group which may be substituted;
B is a single bond; and
Z is a substituted aryl group and the substituent is a group of formula:

$$-G-E$$

wherein G is an oxygen atom or an alkylene group which may be substituted and E is an aryl group which may be substituted.

**17.** An aminopyrimidine derivative or a pharmacologically acceptable salt thereof according to claim 13, wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

$$-A-Y-B-Z \qquad (II)$$

wherein:
A is a single bond;
Y is an alkylene group which may be substituted;
B is an oxygen atom; and
Z is an aryl group which may be substituted.

**18.** An aminopyrimidine derivative or a pharmacologically acceptable salt thereof according to claim 13, wherein in the general formula (I):

$R^6$ is a group of general formula (II):

$$-A-Y-B-Z \qquad (II)$$

wherein:
A is a carbonyl group;
Y is an alkylene group which may be substituted;
B is an oxygen atom or a single bond; and

Z is an aryl group which may be substituted or a heterocyclic group which may be substituted.

**19.** An aminopyrimidine derivative or a pharmacologically acceptable salt thereof according to claim 13, wherein in the general formula (I):

$R^1$ is a hydrogen atom or an alkyl group which may be substituted;
$R^2$ and $R^3$, which may be the same or different, represent a hydrogen atom or an alkyl group which may be substituted, or $R^2$ and $R^3$ may be combined together to form a C3 to C10 alkylene group which may be substituted;
X is a nitrogen atom or a group of C-H;
$R^5$ is a hydrogen atom; and
$R^6$ is a group of general formula (II):

$$-A-Y-B-Z \quad (II)$$

wherein:
A is a carbonyl group;
Y is an alkylene group which may be substituted;
B is an oxygen atom or a single bond; and
Z is an aryl group which may be substituted.

**20.** An aminopyrimidine derivative selected from the group consisting of the compounds below, or a pharmacologically acceptable salt thereof:

N-[2-(2,4-dimethylphenoxy)ethyl]-5,6-dimethyl-[1,2,4]triazolo[1,5-a]pyrimidin-7-amine;
N-{3-[4-(tert-butyl)phenoxy]propyl}-5,6-dimethyl-[1,2,4]triazolo[1,5-a]pyrimidin-7-amine;
5,6-dimethyl-N-{2-[4-(1-methyl-1-phenylethyl)phenoxy]-ethyl}[1,2,4]triazolo[1,5-a]pyrimidin-7-amine;
N-[2-{2,3-dimethyl-4-[4-(methylsulfanyl)phenoxy]-phenoxy}ethyl]-5,6-dimethyl[1,2,4]triazolo[1,5-a]pyrimidin-7-amine;
N-{2-[4-(2-chloro-4-fluorobenzyl)phenoxy]ethyl}-5,6-dimethyl[1,2,4]triazolo[1,5-a]pyrimidin-7-amine;
5,6-dimethyl-N-[2-(2-methyl-4-phenoxyphenoxy)ethyl]-[1,2,4]triazolo[1,5-a]pyrimidin-7-amine;
N-[2-(4-benzyl-2-ethylphenoxy)ethyl]-5,6-dimethyl-[1,2,4]triazolo[1,5-a]pyrimidin-7-amine;
N-[2-(2,4-dimethylphenoxy)ethyl]-5,6,7,8-tetrahydro-[1,2,4]triazolo[5,1-b]quinazolin-9-amine;
N-[2-([1,1'-biphenyl]-4-yloxy)ethyl]-5,6-dimethyl-[1,2,4]triazolo[1,5-a]pyrimidin-7-amine;
N-{2-[4-(2,4-dichlorobenzyl)phenoxy]ethyl}-5,6-dimethyl[1,2,4]triazolo[1,5-a]pyrimidin-7-amine;
N-{2-[4-(benzyloxy)phenoxy]ethyl}-5,6-dimethyl-[1,2,4]triazolo[1,5-a]pyrimidin-7-amine;
N-{2-[4-(3,4-dimethylbenzyl)phenoxy]ethyl}-5,6-dimethyl[1,2,4]triazolo[1,5-a]pyrimidin-7-amine;
5-methyl-N-[2-4(phenoxyphenoxy)ethyl][1,2,4]triazolo-[1,5-a]pyrimidin-7-amine;
N-{2-[4-(tert-butyl)phenoxy]ethyl}-5,6-dimethyl-[1,2,4]triazolo[1,5-a]pyrimidin-7-amine;
5,6-dimethyl-N-[2-(4-phenoxyphenoxy)ethyl][1,2,4]-triazolo[1,5-a]pyrimidin-7-amine;
N-[2-(4-benzylphenoxy)ethyl]-5,6-dimethyl[1,2,4]-triazolo[1,5-a]pyrimidin-7-amine;
5,6-dimethyl-N-[2-(1-naphthyloxy)ethyl][1,2,4]triazolo-[1,5-a]pyrimidin-7-amine;
5,6-dimethyl-N-[2-(2-naphthyloxy)ethyl][1,2,4]triazolo-[1,5-a]pyrimidin-7-amine;
N-[3-(4-benzylphenoxy)propyl]-5,6-dimethyl[1,2,4]-triazolo[1,5-a]pyrimidin-7-amine;
N-[2-(2-bromo-4-phenoxyphenoxy)ethyl]-5,6-dimethyl-[1,2,4]triazolo[1,5-a]pyrimidin-7-amine;
N-[2-(2-chloro-4-phenoxyphenoxy)ethyl]-5,6-dimethyl-[1,2,4]triazolo[1,5-a]pyrimidin-7-amine;
N-[2-(4-benzyl-2-chlorophenoxy)ethyl]-5,6-dimethyl-1,2,4]triazolo[1,5-a]pyrimidin-7-amine;
N-[2-(2,6-dimethyl-4-phenoxyphenoxy)ethyl]-5,6-dimethyl[1,2,4]triazolo[1,5-a]pyrimidin-7-amine;
N-(2-{2,6-dimethyl-4-[4-(methylsulfanyl)phenoxy]-phenoxy}ethyl)-5,6-dimethyl[1,2,4]triazolo[1,5-a]pyrimidin-7-amine;
N-{2-[4-(2,4-dimethylphenoxy)-2,6-dimethylphenoxy]-ethyl}-5,6-dimethyl[1,2,4]triazolo[1,5-a]pyrimidin-7-amine;
5,6-dimethyl-N-(2-{4-[4-(methylsulfanyl)phenoxy]-phenoxy}ethyl)[1,2,4]triazolo[1,5-a]pyrimidin-7-amine;
N-[2-(2-fluoro-4-phenoxyphenoxy)ethyl]-5,6-dimethyl-[1,2,4]triazolo[1,5-a]pyrimidin-7-amine;
N-[2-(2,3-dimethyl-4-phenoxyphenoxy)ethyl]-5,6-dimethyl[1,2,4]triazolo[1,5-a]pyrimidin-7-amine;
5,6-dimethyl-N-{2-[4-(phenylsulfanyl)phenoxy]ethyl}-[1,2,4]triazolo[1,5-a]pyrimidin-7-amine;
N-[2-(4-benzyl-2,3-dimethylphenoxy)ethyl]-5,6-dimethyl-[1,2,4]triazolo[1,5-a]pyrimidin-7-amine;

5,6-dimethyl-N-{2-[4-(4-methylbenzyl)phenoxy]ethyl}-[1,2,4]triazolo[1,5-a]pyrimidin-7-amine;
N-{2-[4-(2-fluorobenzyl)phenoxy]ethyl}-5,6-dimethyl-[1,2,4]triazolo[1,5-a]pyrimidin-7-amine;
5,6-dimethyl-N-(2-{4-[4-(methylsulfanyl)benzyl]-phenoxy}ethyl)[1,2,4]triazolo[1,5-a]pyrimidin-7-amine;
5,6-dimethyl-N-{2-[4-(1-phenylethyl)phenoxy]ethyl}-[1,2,4]triazolo[1,5-a]pyrimidin-7-amine;
N-[2-(4-benzyl-2-methylphenoxy)ethyl]-5,6-dimethyl-[1,2,4]triazolo[1,5-a]pyrimidin-7-amine;
N-[2-(4-benzylphenoxy)ethyl]-5-methyl[1,2,4]triazolo-[1,5-a]pyrimidin-7-amine;
N-[2-(4-benzyl-2-methylphenoxy)ethyl]-5-methyl[1,2,4]-triazolo[1,5-a]pyrimidin-7-amine;
N-[2-(2-methyl-4-phenoxyphenoxy)ethyl]-N-(5-methyl-[1,2,4]triazolo[1,5-a]pyrimidin-7-yl)amine;
N-[2-(4-benzyl-2-methylphenoxy)ethyl]-5-ethyl[1,2,4]-triazolo[1,5-a]pyrimidin-7-amine;
N-[2-(4-benzyl-2-ethylphenoxy)ethyl]-5-methyl[1,2,4]-triazolo[1,5-a]pyrimidin-7-amine;
N-[2-(4-benzyl-2,6-dimethylphenoxy)ethyl]-5,6-dimethyl-[1,2,4]triazolo[1,5-a]pyrimidin-7-amine;
N-[2-(4-benzyl-2,6-dimethylphenoxy)ethyl]-5-methyl-[1,2,4]triazolo[1,5-a]pyrimidin-7-amine;
N-[2-(4-benzyl-2-methylphenoxy)ethyl][1,2,4]triazolo-[1,5-a]pyrimidin-7-amine;
5,6-dimethyl-N-{4-[4-(methylsulfanyl)phenoxy]benzyl}-[1,2,4]triazolo[1,5-a]pyrimidin-7-amine;
5,6-dimethyl-N-{4-[4-(methylsulfinyl)phenoxy]benzyl}-[1,2,4]triazolo[1,5-a]pyrimidin-7-amine;
5,6-dimethyl-N-{4-[4-(methylsulfonyl)phenoxy]benzyl}-[1,2,4]triazolo[1,5-a]pyrimidin-7-amine;
N-[2-(2,4-dimethylphenoxy)ethyl]-5,6-dimethylpyrazolo-[1,5-a]pyrimidin-7-amine;
N-{2-[4-(tert-butyl)phenoxy]ethyl}-5,6-dimethylpyrazolo[1,5-a]pyrimidin-7-amine;
N-[2-(4-benzyl-2-methylphenoxy)ethyl]-5,6-dimethylpyrazolo[1,5-a]pyrimidin-7-amine;
5,6-dimethyl-N-[2-(2-methyl-4-phenoxyphenoxy)ethyl]-pyrazolo[1,5-a]pyrimidin-7-amine;
N-[2-(4-benzyl-2,3-dimethylphenoxy)ethyl]-5,6-dimethylpyrazolo[1,5-a]pyrimidin-7-amine;
N-[2-(4-benzyl-2-methylphenoxy)ethyl]pyrazolo[1,5-a]pyrimidin-7-amine; and
N-[2-(4-benzylphenoxy)ethyl]-2-(tert-butyl)-5-methylpyrazolo[1,5-a]pyrimidin-7-amine.

**21.** An aminopyrimidine derivative or a pharmacologically acceptable salt thereof according to any one of claims 13 through 20 for use as an active ingredient of a pharmaceutical composition.

**22.** Use of an aminopyrimidine derivative or a pharmacologically acceptable salt thereof according to any one of claims 1 through 20 in the production of a pharmaceutical composition for suppressing fat accumulation.

**23.** A method for suppressing the accumulation of fat which comprises administering to a subject of interest an effective dose of an aminopyrimidine derivative or a pharmacologically acceptable salt thereof according to any one of claims 1 through 20.

**24.** A method for preparing a mature adipocyte population which comprises bringing a substance having prostaglandin $J_2$ activity and a differentiation inducing substance in contact with a confluent preadipocyte population, which is separated from animal adipose tissues and has a population doubling level of not greater than 4 after the separation, followed by incubation.

**25.** A method for preparing a mature adipocyte population according to claim 24, wherein the animal is a mammal.

**26.** A method for preparing a mature adipocyte population according to claim 24 or 25, wherein the animal is a rat.

**27.** A method for preparing a mature adipocyte population according to any one of claims 24 to 26, wherein the adipose tissues are adipose tissues in the vicinity of mesenterium.

**28.** A method for assaying a fat accumulation suppressing activity of a test substance which comprises culturing a mature adipocyte population prepared by the method according to any one of claims 24 to 27 in contact with a test substance or a control substance, measuring a fat content of the cell population cultured, and determining a level of fat accumulation suppression in the mature adipocyte population cultured in contact with the test substance, based on a difference between the fat content in the cell population cultured in contact with the test substance and the fat content in the cell population cultured in contact with the control substance.

**29.** A method for assaying a fat accumulation suppressing activity of a test substance according to claim 28, wherein the cultured mature adipocyte population is stained with oil red O and the fat content in the cell population is measured based on the staining intensity.

**30.** A method for screening a substance capable of suppressing the accumulation of fat, which comprises using the

assay method according to claim 28 or 29 to determine a suppression level of fat accumulation in the mature adipocyte population cultured in contact with a test substance and selecting the test substance in terms of a degree of the suppression.

**31.** A substance capable of suppressing the accumulation of fat which is selected by the screening method according to claim 30.

**32.** A pharmaceutical composition for suppressing the accumulation of fat comprising as an active ingredient a substance capable of suppressing the accumulation of fat according to claim 31.

## INTERNATIONAL SEARCH REPORT

International application No.
PCT/JP00/00462

A. CLASSIFICATION OF SUBJECT MATTER
Int.Cl[7] C07D487/04, A61K31/519, A61P3/04, 3/06, 3/10, C12N5/06, C12Q1/02

According to International Patent Classification (IPC) or to both national classification and IPC

B. FIELDS SEARCHED

Minimum documentation searched (classification system followed by classification symbols)
Int.Cl[7] C07D487/04, A61K31/519, A61P3/04, 3/06, 3/10, C12N5/06, C12Q1/02

Documentation searched other than minimum documentation to the extent that such documents are included in the fields searched

Electronic data base consulted during the international search (name of data base and, where practicable, search terms used)
CA(STN)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP, 4-84134, A2 (Konica Corporation), 17 March, 1992 (17.03.92) (Family: none) | 13,18,19 |
| X | JP, 4-76530, A2 (Konica Corporation), 11 March, 1992 (11.03.92) (Family: none) | 13,18,19 |
| X | JP, 45-30335, B4 (Shionogi & Co., Ltd.), 01 October, 1970 (01.10.70) (Family: none) | 13,18 |
| A | JP, 10-182461, A2 (NIPPON CHEMIPHAR CO., LTD.), 07 July, 1998 (07.07.98) & WO, 9820871, A1 | 1-22,24-30 |
| A | JP, 7-309765, A2 (Fujisawa Pharmaceutical Co., Ltd.), 28 November, 1995 (28.11.95) (Family: none) | 1-22,24-30 |
| A | CH.GIESSLEN,A.FALN,V.V.TERTOV,S.A.KUDRYASHOV,A.N,OREKHOV,V.N.SMIRNOV,H.J.MEST, "Trapidil Derivatives as Potential Antiatherosclerotic Drugs", Arzneimittel-Gorschung, 1987, Vol.37, No.5,p.538-541 | 1-22,24-30 |
| X | MARIA ADAMS, CARL T. MONTAGUE, JOHANNES B. PRINS, JULIE | 24-30 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 April, 2000 (10.04.00) | 25 April, 2000 (25.04.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP00/00462

C (Continuation). DOCUMENTS CONSIDERED TO BE RELEVANT

| Category* | Citation of document, with indication, where appropriate, of the relevant passages | Relevant to claim No. |
|---|---|---|
| Y | C.HOLDER, STEPHEN A. SMITH, LOUISE SANDERS, JAN E. DIGBY, CLARAN P. SEWTER, MITCHELL A. LAZAR, V.KRISHNA K.CHATTERJEE, STEPHEN O'RAHILLY," Activators of Peroxisome Proliferator-activated Receptor gamma Have Depot-specific Effects on Human Preadipocyte Differentiation", Journal of Clinical Investigation, 1997, Vol.100, No.12, p.3149-3153 | |
| Y | ZHIDAN WU, YUHONG XIE, NANCY L. R. BUCHER, STEPHEN R. FARMER, "Conditional ectopic expression of C/EBPbeta in NIH-3T3 cells induces PPARgamma and stimulates adipogenesis", Genes & Development, 1995, Vol.9, No.19, p.2350-2363 | 24-30 |

Form PCT/ISA/210 (continuation of second sheet) (July 1992)

**INTERNATIONAL SEARCH REPORT**

International application No.
PCT/JP00/00462

**Box I Observations where certain claims were found unsearchable (Continuation of item 1 of first sheet)**

This international search report has not been established in respect of certain claims under Article 17(2)(a) for the following reasons:

1. ☒ Claims Nos.: 23
because they relate to subject matter not required to be searched by this Authority, namely:

The subject matter of claim 23 relates to a method for treatment of the human body by therapy.

2. ☒ Claims Nos.: 31,32
because they relate to parts of the international application that do not comply with the prescribed requirements to such an extent that no meaningful international search can be carried out, specifically:

It can never be technically understood what kind of chemicals the fat accumulation inhibitors are (chemical structure, physical properties, characteristics, etc. for specifying chemicals are unknown). Thus, International Search cannot be practiced.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The all inventions as set forth in claims have no technical feature.

The chemical structure of the all compounds as set forth in claims 13 to 20 is not a novel one. Also, the "fat accumulation inhibition", i.e., the technical feature of the all inventions as set forth in claims 1 to 12, 21, 22 and 24 to 32 is not a novel one. Therefore, they cannot be regarded as any technical feature.

Such being the case, the present international application has six groups of inventions, i.e., "fat accumulation inhibitory agents containing compounds of the formula (I)", "compounds of the formula (I) wherein the substituent $R^6$ is -CO-Y-B-Z", "compounds of the formula (I) wherein the substituent $R^6$ is -single bond-Y-O-Z", "compounds of the formula (I) wherein the substituent $R^6$ is -single bond-Y-single bond-Z-G-E", "method for preparing matured fat cells and measurement method with the use thereof" and "fat accumulation inhibitors and fat accumulation inhibitory agents containing the same".

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1992)